(19) Europäisches Patentamt
European Patent Office
Office européen des brevets

(11) EP 3 689 362 A1

(12) EUROPEAN PATENT APPLICATION

(43) Date of publication:
05.08.2020 Bulletin 2020/32

(21) Application number: 20151685.3

(22) Date of filing: 27.04.2015

(51) Int Cl.:
*A61K 38/00* (2006.01) *A61P 25/00* (2006.01)
*A61K 38/05* (2006.01) *A61K 38/06* (2006.01)
*A61K 9/00* (2006.01) *A61K 47/12* (2006.01)
*A61K 47/02* (2006.01) *A61K 47/18* (2017.01)
*A61K 9/08* (2006.01) *A61K 47/26* (2006.01)
*A61K 31/4025* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2014 US 201461984216 P**

(62) Document number(s) of the earlier application(s) in accordance with Art. 76 EPC:
**15783438.3 / 3 134 103**

(71) Applicant: **Naurex, Inc.**
**Evanston, IL 60201 (US)**

(72) Inventors:
- **Houck, David Renwick**
  **Cary, NC North Carolina 27159 (US)**
- **Arghavani, Mohsen**
  **Lake in the Hills, 60156 (US)**

(74) Representative: **Hoffmann Eitle**
**Patent- und Rechtsanwälte PartmbB**
**Arabellastraße 30**
**81925 München (DE)**

Remarks:
•This application was filed on 14-01-2020 as a divisional application to the application mentioned under INID code 62.
•Claims filed after the date of receipt of the application (Rule 68(4) EPC).

# (54) STABLE COMPOSITIONS OF NEUROACTIVE PEPTIDES

(57) The disclosure relates to intravenous formulations of GLYX peptides for treating CNS Disorders such as depression, neuropathic pain, or anxiety.

EP 3 689 362 A1

SEQUENCE LISTING

<110> NAUREX, INC.

<120> STABLE COMPOSITIONS OF NEUROACTIVE PEPTIDES

<130> NAU-018PC

<140> PCT/US2015/027745
<141> 2015-04-27

<150> 61/984,216
<151> 2014-04-25

<160> 18

<170> PatentIn version 3.5

<210> 1
<211> 11
<212> PRT
<213> Artificial Sequence

<220>
<223> Description of Artificial Sequence: Synthetic peptide

<400> 1
```
Lys Ala Ser Gln Asp Val Ser Thr Thr Val Ala
1               5                   10
```

<210> 2
<211> 7
<212> PRT
<213> Artificial Sequence

<220>
<223> Description of Artificial Sequence: Synthetic peptide

<400> 2
```
Ser Ala Ser Tyr Arg Tyr Thr
1               5
```

<210> 3
<211> 9
<212> PRT
<213> Artificial Sequence

<220>
<223> Description of Artificial Sequence: Synthetic peptide

<400> 3
```
Gln Gln His Tyr Ser Thr Pro Pro Thr
1               5
```

<210> 4
<211> 13
<212> PRT

```
<213> Artificial Sequence

<220>
<223> Description of Artificial Sequence: Synthetic
      peptide

<400> 4
Val Tyr Tyr Ser Gln Gln His Tyr Ser Thr Pro Pro Thr
1               5                   10


<210> 5
<211> 17
<212> PRT
<213> Artificial Sequence

<220>
<223> Description of Artificial Sequence: Synthetic
      peptide

<400> 5
Glu Asp Leu Ala Val Tyr Tyr Ser Gln Gln His Tyr Ser Thr Pro Pro
1               5                   10                  15


Thr



<210> 6
<211> 22
<212> PRT
<213> Artificial Sequence

<220>
<223> Description of Artificial Sequence: Synthetic
      peptide

<400> 6
Ser Val Gln Ala Glu Leu Asp Leu Ala Val Tyr Tyr Ser Gln Gln His
1               5                   10                  15


Tyr Ser Thr Pro Pro Thr
            20


<210> 7
<211> 26
<212> PRT
<213> Artificial Sequence

<220>
<223> Description of Artificial Sequence: Synthetic
      peptide

<400> 7
Phe Thr Ile Ser Ser Val Gln Ala Glu Leu Asp Leu Ala Val Tyr Tyr
1               5                   10                  15


Ser Gln Gln His Tyr Ser Thr Pro Pro Thr
            20                  25
```

<210> 8
<211> 13
<212> PRT
<213> Artificial Sequence

<220>
<223> Description of Artificial Sequence: Synthetic peptide

<400> 8
Gln Gln His Tyr Ser Thr Pro Pro Thr Phe Gly Gly Gly
1 5 10

<210> 9
<211> 17
<212> PRT
<213> Artificial Sequence

<220>
<223> Description of Artificial Sequence: Synthetic peptide

<400> 9
Gln Gln His Tyr Ser Thr Pro Pro Thr Phe Gly Gly Gly Thr Lys Leu
1 5 10 15

Glu

<210> 10
<211> 11
<212> PRT
<213> Artificial Sequence

<220>
<223> Description of Artificial Sequence: Synthetic peptide

<400> 10
Cys Gln Gln His Tyr Ser Thr Pro Pro Thr Cys
1 5 10

<210> 11
<211> 11
<212> PRT
<213> Artificial Sequence

<220>
<223> Description of Artificial Sequence: Synthetic peptide

<400> 11
Ser Gln Gln His Tyr Ser Thr Pro Pro Thr Ser
1 5 10

<210> 12

<211> 5
<212> PRT
<213> Artificial Sequence

<220>
<223> Description of Artificial Sequence: Synthetic peptide

<400> 12
Gln Gln His Tyr Ser
1               5

<210> 13
<211> 4
<212> PRT
<213> Artificial Sequence

<220>
<223> Description of Artificial Sequence: Synthetic peptide

<400> 13
Thr Pro Pro Thr
1

<210> 14
<211> 3
<212> PRT
<213> Artificial Sequence

<220>
<223> Description of Artificial Sequence: Synthetic peptide

<400> 14
Thr Pro Pro
1

<210> 15
<211> 3
<212> PRT
<213> Artificial Sequence

<220>
<223> Description of Artificial Sequence: Synthetic peptide

<400> 15
Pro Pro Thr
1

<210> 16
<211> 2
<212> PRT
<213> Artificial Sequence

<220>
<223> Description of Artificial Sequence: Synthetic peptide

<400> 16
Pro Pro
1

<210> 17
<211> 3
<212> PRT
<213> Artificial Sequence

<220>
<223> Description of Artificial Sequence: Synthetic peptide

<400> 17
Thr Pro Thr
1

<210> 18
<211> 1
<212> PRT
<213> Artificial Sequence

<220>
<223> Description of Artificial Sequence: Synthetic peptide

<400> 18
Thr
1

## Description

CROSS-REFERENCE TO RELATED APPLICATIONS

This application claims the benefit of United States Provisional Application No. 61/984,216, filed on April 25, 2014, which is incorporated herein by reference in its entirety.

BACKGROUND

The central nervous system (CNS) of mammals employs many neuroactive peptides to effect specialized signaling within the brain and spinal cord including the neuroactive peptides somatostatin, cholecystokinin, VIP, Substance P, enkephalin, Neuropeptide Y (NPY), Neurotensin, TRH, CCK, and dynorphin. (The Biochemical Basis of Neuropharmacology, Cooper, Bloom and Roth, 5th ed., Oxford University Press, New York, 1986). The careful elucidation of the complex signaling pathways, which operate in the CNS, has led to identification of specific receptors modulated by these neuroactive peptides presenting important therapeutic targets for various disorders associated with the CNS.

An N-methyl-D-aspartate (NMDA) receptor (NMDAR) is one such receptor that has drawn particular interest since it appears to be involved in a broad spectrum of CNS disorders. The NMDAR has been implicated in neurodegenerative disorders including stroke-related brain cell death, convulsive disorders, and learning and memory. NMDAR also plays a central role in modulating normal synaptic transmission, synaptic plasticity, and excitotoxicity in the central nervous system. The NMDAR is further involved in Long-term potentiation (LTP). LTP is the persistent strengthening of neuronal connections that underlie learning and memory (See Bliss and Collingridge, 1993, Nature 361:31-39).

Several compounds have been identified which exert a dual (agonist/antagonist) effect on the NMDA receptor through the allosteric sites. These compounds are termed "partial agonists". In the presence of the principal site ligand, a partial agonist will displace some of the ligand and thus decrease $Ca^{++}$ flow through the receptor. In the absence of or lowered level of the principal site ligand, the partial agonist acts to increase $Ca^{++}$ flow through the receptor channel.

Recently, an improved partial agonist of NMDAR, termed as GLYX-13 or rapastinel, has been reported. GLYX-13 is exemplified by the following structure:

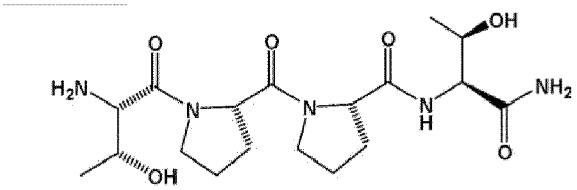

,

with a molecular weight: 413.47, and a chemical formula: $C_{18}H_{31}N_5O_6$. GLYX-13 exhibits nootropic, neuroprotective and antinociceptive activity, and enhances learning, memory and cognition in vivo.

Although a number of therapeutic benefits of GLYX-13 have already been elucidated, there still remains a need for ways to efficiently deliver GLYX-13 so as to ensure GLYX-13 effectively crosses the blood brain barrier and is efficiently absorbed at the required site of action to effect improved treatment of CNS disorders like depression, neuropathic pain, or anxiety. Additionally, it would be desirable that the delivery formulation is stable (i.e., not subject to degradation) in an aqueous media.

SUMMARY

This disclosure features stable compositions (e.g., aqueous compositions) that are suitable for intravenous injection and that include a GLYX peptide and/or a derivative and/or salt thereof (e.g., a GLYX-13 peptide and/or derivative and/or salt thereof; e.g., a GLYX peptide having the following formula:

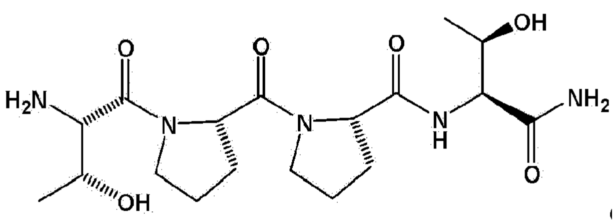

or a salt thereof). Embodiments can include one or more of the following advantages. In some embodiments, the compositions described herein can enhance clinical delivery or administration of the GLYX peptide (e.g., a GLYX-13 peptide and/or derivative and/or salt thereof; e.g., a GLYX peptide having the formula shown above or salt thereof) to the circulatory system by IV injection, thereby resulting in more efficient delivery of the GLYX peptide to the brain, and thus to one or more active sites in the brain associated with treatment of CNS disorders such as depression, neuropathic pain, or anxiety. In some embodiments, the compositions described herein can exhibit enhanced storage stability, e.g., rendering the GLYX peptides less susceptible to degradation in aqueous media. The compositions described herein can further include one or more pharmaceutically acceptable moieties (e.g., including but not limited to, a buffer, a water miscible solvent, an excipient, pharmaceutically acceptable anions (e.g., chloride ion) and cations (e.g., $H^+$)) that confer one or more physical and/or chemical properties to the compositions. For example, the one or more pharmaceutically acceptable moieties can be an acid (e.g., hydrochloric acid, e.g., dissociated hydrochloric acid), its conjugate base (sometimes referred to herein as a "buffer," e.g., chloride ion), or a combination thereof and can be present in amounts sufficient to maintain a particular pH. It will also be appreciated by those skilled in the art that the compositions described herein may additionally contain, if desired, a combination of two or more active additional ingredients.

In one aspect, this disclosure features a stable, aqueous composition suitable for intravenous injection, comprising a compound represented by:

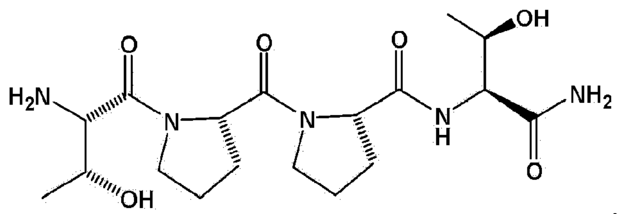

,

or a pharmaceutically acceptable salt thereof. Such compositions can further include one or more acids, one or more buffers and /or one or more excipients.

In one aspect, this disclosure features a stable, aqueous composition suitable for intravenous injection, comprising: (i) 60 mg/mL to about 200 mg/mL (e.g., about 125 mg/mL to about 175 mg/mL; e.g., about 150 mg/mL or about 75 mg/mL) of a pharmaceutically active compound having the formula:

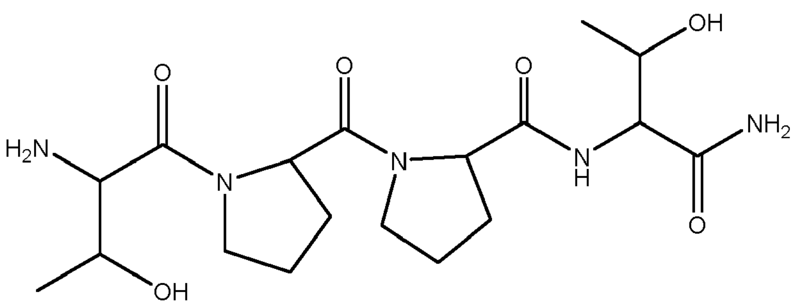

;

or a pharmaceutically acceptable salt thereof; (ii) water for injection; and (iii) an acid; wherein the stable, aqueous composition has a pH of from about 3.9 to about 5.5 at 25 °C.

In another aspect, this disclosure features a receptacle (e.g., a prefilled syringe or vial) containing an amount of any of the stable, aqueous compositions described herein. In certain embodiments, the amount is extractable as at least one single dose. In certain embodiments, the single dose can have a volume of about 1 mL to about 4 mL (e.g., 3 mL).

In a further aspect, this disclosure features a pre-filled syringe that includes a single dose of any of the stable, aqueous compositions described herein. In certain embodiments, the single dose can have a volume of about 1 mL to about 4 mL (e.g., 3 mL).

In one aspect, this disclosure features a composition that includes: (i) about 150 mg/mL of a compound represented by:

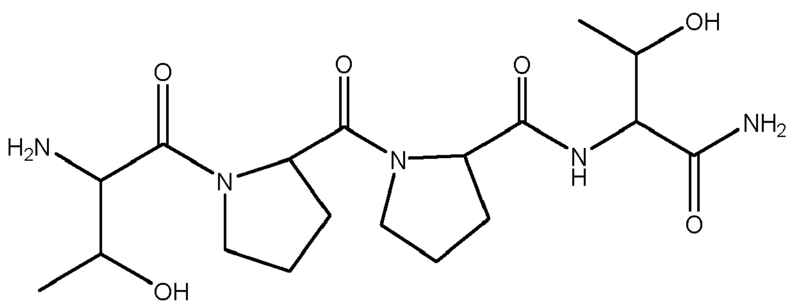

;

(ii) water for injection; and (iii) and hydrochloric acid, wherein the composition has a pH of about 4.1 to about 4.7 at 25 °C.

In one aspect, this disclosure features a pharmaceutically acceptable dose suitable for injection comprising: (i) about 450 mg of a compound represented by:

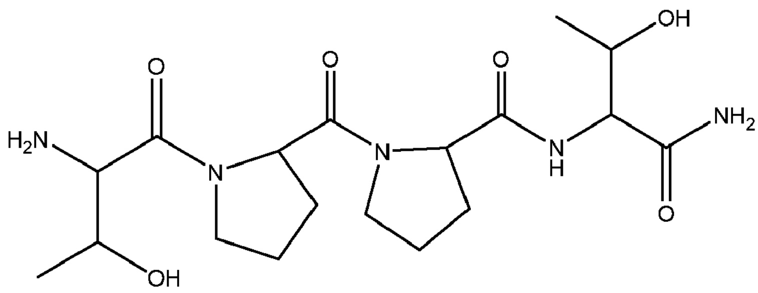

;

(ii) water; and an acid providing chloride ions in the aqueous composition, wherein the dose has a pH of about 4.5 and a volume of about 3 mL. In certain embodiments, the dose can be disposed within a syringe or a vial.

In one aspect, this disclosure features a pharmaceutically acceptable dose suitable for injection comprising: (i) about 225 mg of a compound represented by:

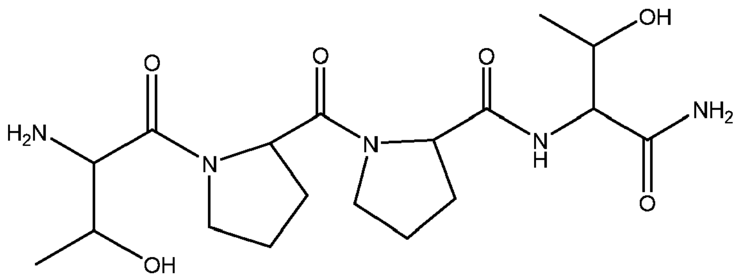

;

water for injection; and hydrochloric acid, wherein the does has a pH of about 4.5 and a volume of about 3 mL. In certain embodiments, the dose can be disposed within a syringe or a vial.

In another aspect, this disclosure features a stable, aqueous composition suitable for intravenous injection, comprising from about 100 mg/mL to about 200 mg/mL (e.g., from about 125 mg/mL to about 175 mg/mL, from about 140 mg/mL to about 160 mg/mL, or about 150 mg/mL) of a compound having the formula:

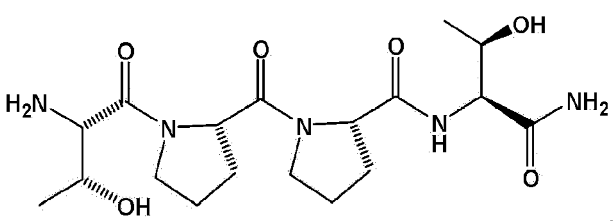

,

or a pharmaceutically acceptable salt thereof; wherein the composition has a pH of from about 3.5 to about 6.5 (e.g.,

from about 3.5 to about 5.5, from about 3.9 to about 5.5, from about 4.0 to about 5.0, from about 4.2 to about 5.0, from about 4.2 to about 4.8, about 4.0, about 4.5) at 25 °C. In some embodiments, the aqueous composition can further comprise an acid (e.g., hydrochloric acid) and/or buffer (e.g., chloride ion) and/or one or more excipients.

Embodiments can include one or more of the following features. The stable, aqueous composition can include about 200 mg to about 500 mg (e.g., about 450 mg; about 375; or about 225 mg) of the pharmaceutically active compound. The stable, aqueous composition can have a pH of about 4.5 at 25 °C. The stable, aqueous composition can include at least one of: $H^+$, a protonated form of the pharmaceutically active compound, and/or a combination thereof. The acid can be selected from the group consisting of fumaric acid, malic acid, lactic acid, hydrochloric acid, hydrobromic acid, acetic acid, citric acid, phosphoric acid, nitric acid, sulfuric acid, and ascorbic acid. In certain embodiments, the acid provides chloride ions in the aqueous composition (e.g., .hydrochloric acid). In certain embodiments, upon administration of a dose of the stable, aqueous liquid composition that comprises about 150 mg/mL of the pharmaceutically active compound and has a volume of about 3 mL to a patient, a physiological osmolality of from about 800 mOsmol/kg to about 900 mOsmol/kg is obtained in said patient. In other embodiments, upon administration of a dose of the stable, aqueous liquid composition that comprises about 75 mg/mL of the pharmaceutically active compound and has a volume of about 3 mL to a patient, a physiological osmolality of from about 375 mOsmol/kg to about 475 mOsmol/kg is obtained in said patient. The composition can have a minimal amount of one or more of degradation products each selected from the group consisting of cyclo proline-threonine (diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature or after 20 days at room temperature. The composition can have a minimal amounts of one or more of degradation products each selected from the group consisting of diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 month at 0 °C or below. The composition can have less than about 2% area obtained by HPLC of the GLYX -13 peak of diketopiperazine and/or Pro-Thr-$NH_2$ after 3 months at 40 °C. The composition can have less than about 1% or less than about 0.5% area obtained by HPLC of the GLYX -13 peak by HPLC of diketopiperazine and/or Pro-Thr-$NH_2$ after 3 weeks at 40 °C. The composition can be prepared by a process that includes: (i) providing a first combination comprising the pharmaceutically active compound and water; and (ii) contacting the first combination with hydrochloric acid, or a source thereof, in an amount sufficient to achieve a pH of from about 3.9 to about 5.5.

Embodiments can include one or more of the following features. The composition can further include an acid, which is selected from the group consisting of hydrochloric acid, phosphoric acid, and sulfuric acid. The composition can further include a buffer. In certain embodiments, the buffer is selected from the group consisting of chloride ion, sodium ion, potassium ion, and ammonium ion. In other embodiments, the buffer is selected from the group consisting of acetate, citrate, phosphate, succinic, carbonate, bicarbonate, and maleic acid and salts thereof. The composition can have a pH of from about 3.9 to about 5.5 (e.g., 4.5) at 25 °C. In certain embodiments, upon administration of the composition, a physiological osmolality of from about 500 mOsmol/kg to about 1,000 mOsmol/kg (e.g., 600 mOsmol/kg to about 950 mOsmol/kg or 850 mOsmol/kg to about 950 mOsmol/kg) is obtained. The composition can have minimal amounts of one or more of degradation products each selected from the group consisting of cyclo-proline-threonine (i.e. diketopiperazine), proline-threonine-diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature. The composition can have minimal amounts of one or more of degradation products each selected from the group consisting of cyclo-proline-threonine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 20 days at room temperature. The composition can have minimal amounts of one or more of degradation products each selected from the group consisting of cyclo-proline-threonine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 month at 0 °C or below. The composition can have minimal amounts of diketopiperazine and/or Pro-Thr-$NH_2$.

In a further aspect, this disclosure features a prefilled syringe comprising a stable, aqueous composition having from about 350 mg to about 800 mg (e.g., from about 400 mg to about 750 mg, from about 350 mg to about 500 mg, from about 350 mg to about 450 mg, from about 400 mg to about 500 mg, about 400 mg, about 450 mg, from about 600 mg to about 800 mg, from about 700 mg to about 800 mg, about 750 mg) of a compound represented by:

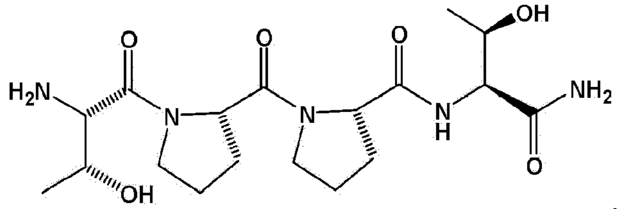

,

or a pharmaceutically acceptable salt thereof; in from about 2 mL to about 6 mL (e.g., from about 2.5 mL to about 5 mL, from about 2 mL to about 4 mL, from about 2.5 mL to about 3.5 mL, about 2.7 mL, about 3 mL, from about 4 mL to about 6 mL, from about 4.5 mL to about 5.5 mL, about 5mL) of an aqueous solution. In some embodiments, the aqueous composition can further comprise an acid (e.g., hydrochloric acid) and/or buffer (e.g., chloride ion) and/or one or more excipients.

Embodiments can include one or more of the following features. In certain embodiments, the aqueous composition can have from about 400 mg to about 750 mg of the compound or salt and, optionally, have from about 2.5 mL to about 5 mL of an aqueous solution. In certain embodiments, the aqueous composition can have from about 350 mg to about 500 mg (e.g., from about 350 mg to about 450 mg or from about 400 mg to about 500 mg) of the compound or salt and, optionally, have from about 2 mL to about 4 mL of an aqueous solution. For example, the aqueous composition can have about 400 mg of the compound or salt in about 2.7 mL of an aqueous solution. As another example, the aqueous composition can have about 450 mg of the compound or salt in about 3 mL of an aqueous solution. In other embodiments, the aqueous composition can have from about 600 mg to about 800 mg (e.g., from about 700 mg to about 800 mg) of the compound or salt and, optionally, have from about 4 mL to about 6 mL of an aqueous solution. For example, the aqueous composition can have about 750 mg of the compound or salt in about 5 mL of an aqueous solution. The aqueous composition has a pH of from about 3.9 to about 5.5 (e.g., 4.5) at 25 °C. The composition can have minimal amounts of one or more of degradation products each selected from the group consisting of diketopiperazine, proline-threonine-diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature. The composition can have minimal amounts of one or more of degradation products each selected from the group consisting of proline-threonine-diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 20 days at room temperature. The composition can have minimal amounts of one or more of degradation products each selected from the group consisting of diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 month at 0 °C or below. The composition can have minimal amounts of proline-threonine-diketopiperazine and/or Pro-Thr-$NH_2$.

In one aspect, the disclosure provides a composition comprising a compound represented by the formula:

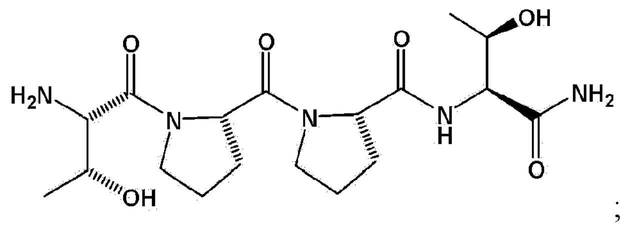

;

water (e.g., water for injection); and chloride ion, in which the composition has a pH of from about 3.5 to about 6.5 (e.g., from about 3.5 to about 5.5, from about 3.9 to about 5.5, from about 3.5 to about 4.5, from about 4.0 to about 5.0, from about 4.2 to about 5.0, from about 4.2 to about 4.8, about 4.0, about 4.5 at room temperature). Embodiments can include one or more of the following features. The composition can include from about 100 mg/mL to about 200 mg/mL (e.g., 150 mg/mL) of the compound. In certain embodiments, less than 2% of the compound is degraded after 21 days at 40°C. In certain embodiments, the composition has minimal amounts of diketopiperazine. In other embodiments, the composition has minimal amounts of proline-threonine amide. The water can be water for injection. The cationic counterion can be $H^+$, a protonated form of the compound, or a combination thereof.

In another aspect, the disclosure provides a composition consisting essentially of, and/or consisting of a compound represented by the formula:

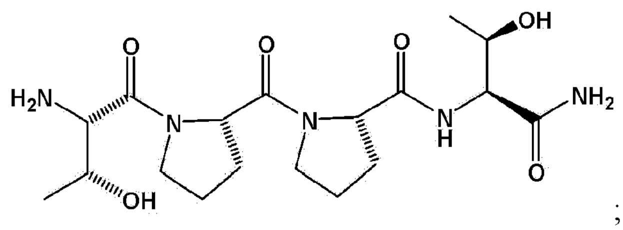

;

water (e.g., water for injection); and chloride ion, in which the composition has a pH of from about 3.5 to about 6.5 (e.g., from about 3.5 to about 5.5, from about 3.9 to about 5.5, from about 3.5 to about 4.5, from about 4.0 to about 5.0, from

about 4.2 to about 5.0, from about 4.2 to about 4.8, about 4.0, about 4.5 at room temperature). Embodiments can include one or more of the following features. The composition can include from about 100 mg/mL to about 200 mg/mL (e.g., 150 mg/mL) of the compound. In certain embodiments, less than 2% of the compound is degraded after 21 days at 40°C. In certain embodiments, the composition has minimal amounts of diketopiperazine. In other embodiments, the composition has minimal amounts of proline-threonine amide. The water can be water for injection. The cationic counterion can be $H^+$, a protonated form of the compound, or a combination thereof.

In one aspect, the disclosure provides a stable, aqueous composition suitable for intravenous injection, comprising: a compound represented by the formula:

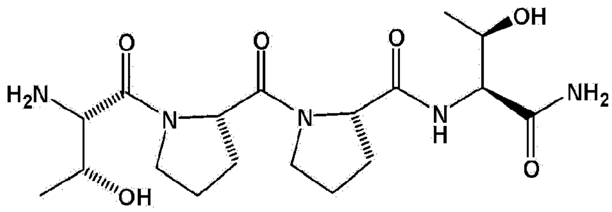

or a pharmaceutically acceptable salt thereof; and about 0.5 to about 1.2 mole percent (e.g., about 0.6 to about 1.0 mole percent) of a salt such as sodium chloride in an aqueous solution.

In another aspect, the disclosure provides a stable, aqueous composition suitable for intravenous injection, comprising: a compound represented by the formula:

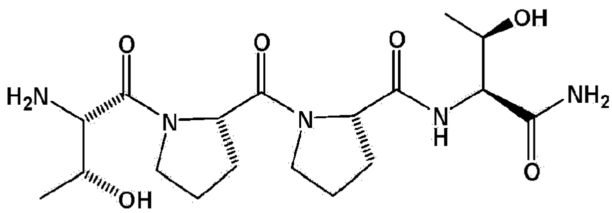

or a pharmaceutically acceptable salt thereof; a buffer; and optionally a water-miscible cosolvent.

Embodimets can include one or more of the following features. In certain embodiments, the buffer can be selected from the group consisting of acetate, citrate, phosphate, sulfate, succinate, carbonate, bicarbonate, arginine, and maleic acid and salts thereof. In other embodiments, the buffer can be an anion selected from the group consisting of chloride, sulfate, and phosphate. In still other embodiments, the buffer is citric acid. The cosolvent can be selected from the group consisting of polyethylene glycol, glycerine, ethanol, polypropylene glycol, and N,N-diemethylacetamide (e.g., polyethylene glycol having a molecular weight of about 200 to about 900Da or polyethylene glycol having a molecular weight of about 400Da). The pH of the composition can be about 4.5 to about 6.0 at 25°C. The composition can have less than 0.5 weight percent of diketopiperazine or Pro-Thr-$NH_2$ after 10 days (e.g., less than 0.1 weight percent diketopiperazine or Pro-Thr-$NH_2$ after 10 days). The composition can have less than 0.5 weight percent Thr-Pro-Pro-$NH_2$ after 10 days (e.g., less than 0.1 weight percent Thr-Pro-Pro-$NH_2$ after 10 days).

BRIEF DESCRIPTION OF THE FIGURES

Figure 1 shows the formation of impurity (% area proline-threonine diketopiperazine, RRT 0.43) over time versus pH of a disclosed composition

Figure 2 shows the formation of impurity (% area proline-threonine-amide, RRT 0.57) over time versus pH of a disclosed composition.

DETAILED DESCRIPTION

This disclosure is directed in part to stable compositions (e.g., aqueous compositions) that are suitable for intravenous injection and that include a GLYX peptide and/or a derivative and/or salt thereof (e.g., a GLYX-13 peptide and/or derivative and/or salt thereof; e.g., a GLYX peptide having the following formula:

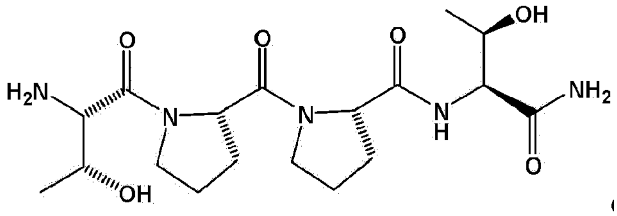

or a pharmaceutically acceptable salt thereof). Embodiments can include one or more of the following advantages. In some embodiments, the compositions described herein can enhance clinical delivery or administration of the GLYX peptide (e.g., a GLYX-13 peptide and/or derivative or salt thereof; e.g., a GLYX peptide having the formula shown above or salt thereof) to the circulatory system by IV injection, thereby resulting in more efficient delivery of the GLYX peptide to the brain, and thus to one or more active sites in the brain associated with treatment of CNS disorders such as depression, neuropathic pain, or anxiety. In some embodiments, the compositions described herein can exhibit enhanced storage stability, e.g., rendering the GLYX peptides less susceptible to degradation in aqueous media. The compositions described herein can, in some embodiments, include one or more pharmaceutically acceptable substances (e.g., including but not limited to, a buffer, a water miscible solvent and/or an excipient) that confer one or more physical and/or chemical properties to the disclosed compositions. For example, the one or more pharmaceutically acceptable substances that may form part of a contemplated composition can be selected from the group consisting of an acid (e.g., hydrochloric acid), its conjugate base (sometimes referred to herein as a "buffer," e.g., chloride ion), or a combination thereof and can be present in amounts sufficient to maintain a particular pH. It will also be appreciated by those skilled in the art that the compositions described herein may additionally contain, if desired, a combination of two or more active additional ingredients.

The chemical and physical stability and/or the pharmaceutical acceptability of disclosed compositions may be determined by techniques well known to those skilled in the art. Thus, for example, the chemical stability of the components may be determined by HPLC assay and/or color by appearance, for example, after prolonged storage of the product. Physical stability data may be gained from other conventional analytical techniques.

The term "about" is used herein to mean approximately, in the region of, roughly, or around. When the term "about" is used in conjunction with a numerical range, it modifies that range by extending the boundaries above and below the numerical values set forth. The term "comprises" is used herein to mean "includes, but is not limited to".

**Peptides**

As used herein, the term "GLYX peptide" refers to a peptide having NMDAR glycine-site partial agonist/antagonist activity. GLYX peptides may be obtained by well-known recombinant or synthetic methods such as those described in US Patents 5,763,393 and 4,086,196 herein incorporated by reference. Exemplary GLYX peptides contemplated as forming part of disclosed formulations may, in some embodiments, include one or more of the listed peptides of Table 1:

**TABLE 1**

| Name SEQ ID NO | Amino Acid Sequence |
|---|---|
| NT-1: SEQ ID. NO:1. | Lys-Ala-Ser-Gln-Asp-Val-Ser-Thr-Thr-Val-Ala |
| NT-2: SEQ ID. NO:2. | Ser-Ala-Ser-Tyr-Arg-Tyr-Thr |
| NT-3: SEQ ID. NO:3. | Gln-Gln-His-Tyr-Ser-Thr-Pro-Pro-Thr |
| NT-4: SEQ ID. NO:4. | Val—Tyr—Tyr—Ser—Gln—Gln—His—Tyr—Ser—Thr—Pro—Pro—Thr |
| NT-5: SEQ ID. NO:5. | Glu—Asp—Leu—Ala—Val—Tyr—Tyr—Ser—Gln—Gln—His—Tyr—Ser—Thr—Pro—Pro—Thr |
| NT-6: SEQ ID. NO:6. | Ser—Val—Gln—Ala—Glu—Leu—Asp—Leu—Ala—Val—Tyr—Tyr—Ser—Gln—Gln—His—Tyr—Ser—Thr—Pro—Pro—Thr |
| NT-7: SEQ ID. NO:7. | Phe—Thr—Ile—Ser—Ser—Val—Gln—Ala—Glu—Leu—Asp—Leu—Ala—Val—Tyr—Tyr—Ser—Gln—Gln—His—Tyr—Ser—Thr—Pro—Pro—Thr |
| NT-8: SEQ ID. NO: 8. | Gln—Gln—His—Tyr—Ser—Thr—Pro—Pro—Thr—Phe—Gly—Gly—Gly |

(continued)

| Name SEQ ID NO | Amino Acid Sequence |
|---|---|
| NT-9: SEQ ID. NO:9. | Gln—Gln—His—Tyr—Ser—Thr—Pro—Pro—Thr—Phe—Gly—Gly—Gly—Thr—Lys—Leu—Glu |
| NT-10: SEQ ID. NO: 10 | 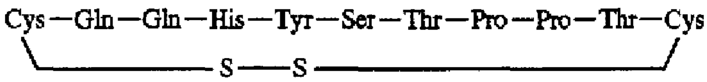 |
| NT-11: SEQ ID. NO:11 | Ser-Gln-Gln-His-Tyr-Ser-Thr-Pro-Pro-Thr-Ser |
| NT-12: SEQ ID. NO:12 | Gln-Gln-His-Tyr-Ser |
| NT-13: SEQ ID. NO:13 | Thr-Pro-Pro-Thr |
| NT-14: SEQ ID. NO:14 | Thr-Pro-Pro |
| NT-15: SEQ ID. NO:15 | Pro-Pro-Thr |
| NT-16: SEQ ID. NO:16 | Pro-Pro |
| NT-17: SEQ ID. NO:17 | Thr-Pro-Thr |
| NT-18: SEQ ID. NO:18 | Thr |

"GLYX-13" is represented by the following formula:

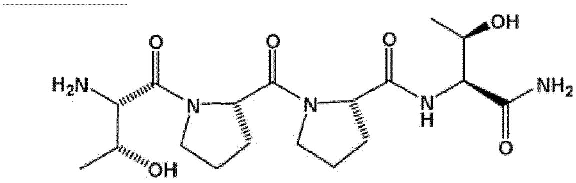

and polymorphs, stereoisomers, hydrates, solvates, free bases, and/or suitable salt forms of the above compound.

For example, disclosed compositions may prove effective in the treatment of many neurological diseases, such as Alzheimer's, Parkinson's, psychiatric diseases and intracerebral infections.

In some embodiments, the compositions described herein have a pH of about 3.5 to about 7 at 25°C. In certain embodiments, the compositions described herein have a pH of from about 4 to about 7 at 25 °C. (e.g., from about 3.5 to about 6.5, from about 3.5 to about 5.5, from about 4 to about 6, from about 3.9 to about 5.5, from about 4.0 to about 5.0, from about 4.2 to about 5.0, from about 4.2 to about 4.8, about 4.0, about 4.5) at 25 °C. In certain embodiments, the compositions described herein have a pH of from 4.2 to about 5.0 (e.g., from about 4.2 to about 4.8, from about 4.3 to about 4.7, from about 4.4 to about 4.6). For example, disclosed compositions in certain embodiments as described herein have a pH of about 4.5.

In some embodiments, the compositions described herein can include less than about 100 mg/mL of a GLYX peptide and/or a derivative and/or salt thereof (e.g., a GLYX-13 peptide and/or derivative and/or salt thereof; e.g., a GLYX peptide having the following formula:

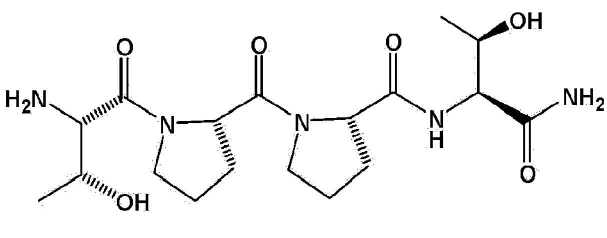

or salt thereof).. In certain embodiments, the compositions described herein include fromabout 25 mg/mL to about 95 mg/mL (e.g., from about 25 mg/mL to about 75 mg/mL, or from about 25 mg/mL to about 55 mg/mL) of the GLYX peptide (e.g., a GLYX-13 peptide and/or derivative thereof; e.g., a GLYX peptide having the formula shown above). In certain embodiments, the compositions described herein include from about 30 mg/mL to about 50 mg/mL of the GLYX peptide (e.g., a GLYX-13 peptide and/or derivative and/or salt thereof; e.g., a GLYX peptide having the formula shown above or salt thereof). In certain embodiments, the compositions described herein include fromabout 35 mg/mL to about 45 mg/mL of the GLYX peptide (e.g., a GLYX-13 peptide and/or derivative and/or salt thereof; e.g., a GLYX peptide having the formula shown above or salt thereof). In other embodiments, the compositions described herein include 60 mg/mL

of the GLYX peptide (e.g., a GLYX-13 peptide and/or derivative and/or salt thereof; e.g., a GLYX peptide having the formula shown above or salt thereof).

In some embodiments, the compositions described herein include from about 100 mg/mL to about 500 mg/mL of a GLYX peptide and/or a derivative and/or salt thereof (e.g., a GLYX-13 peptide and/or derivative and/or salt thereof; e.g., a GLYX peptide having the following formula:

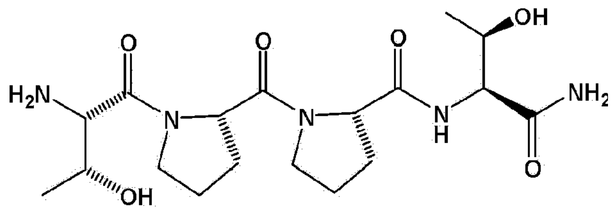

or salt thereof). In certain embodiments, the compositions include from about 100 mg/mL to about 300 mg/mL (e.g., from about 100 mg/mL to about 200 mg/mL, from about 100 mg/mL to about 150 mg/mL of the compound, from about 100 mg/mL to about 125 mg/mL) of the GLYX peptide (e.g., a GLYX-13 peptide and/or derivative and/or salt thereof; e.g., a GLYX peptide having the formula shown above or salt thereof). In certain embodiments, the compositions described herein include from about 100 mg/mL to about 200 mg/mL (e.g., from about 125 mg/mL to about 175 mg/mL, from about 140 mg/mL to about 160 mg/mL, from about 145 mg/mL to about 155 mg/mL,or about 150 mg/mL) of the GLYX peptide (e.g., a GLYX-13 peptide and/or derivative and/or salt thereof; e.g., a GLYX peptide having the formula shown above or salt thereof). In certain embodiments, the compositions described herein include about 150 mg/mL of the the GLYX peptide (e.g., a GLYX-13 peptide and/or derivative and/or salt thereof; e.g., a GLYX peptide having the formula shown above or salt thereof) and have a pH of from about 4 to about 5, e.g., about 4.5, at 25 °C. Such compositions can, for example, be clinically acceptable for IV use (e.g., upon administration of such compositions, a desired physiological osmolality is achieved or maintained; see, e.g., the osmolality parameters described in paragraphs **[0032]** and **[0036]-[0038]**).

In some embodiments, the compositions described herein can have from about 350 mg to about 800 mg (e.g., from about 400 mg to about 750 mg, from about 350 mg to about 500 mg, from about 350 mg to about 450 mg, from about 400 mg to about 500 mg, about 400 mg, about 450 mg, from about 600 mg to about 800 mg, from about 700 mg to about 800 mg, about 750 mg) of a GLYX peptide and/or a derivative and/or salt thereof (e.g., a GLYX-13 peptide and/or derivative and/or salt thereof; e.g., a GLYX peptide having the following formula:

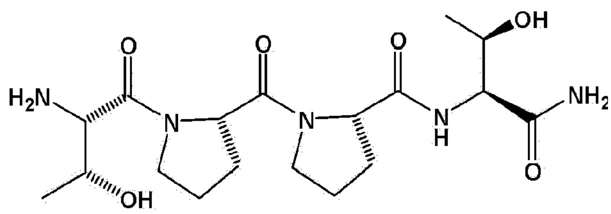

or salt thereof).

In some embodiments, the compositions described herein can have from about 2 mL to about 6 mL (e.g., from about 2.5 mL to about 5 mL, from about 2 mL to about 4 mL, from about 2.5 mL to about 3.5 mL, about 2.7 mL, about 3 mL, from about 4 mL to about 6 mL, from about 4.5 mL to about 5.5 mL, about 5mL) of an aqueous solution.

In some embodiments, the compositions described herein can have from about 350 mg to about 800 mg (e.g., from about 400 mg to about 750 mg, from about 350 mg to about 500 mg, from about 350 mg to about 450 mg, from about 400 mg to about 500 mg, about 400 mg, about 450 mg, from about 600 mg to about 800 mg, from about 700 mg to about 800 mg, about 750 mg) of a GLYX peptide and/or a derivative and/or salt thereof (e.g., a GLYX-13 peptide and/or derivative and/or salt thereof; e.g., a GLYX peptide having the following formula:

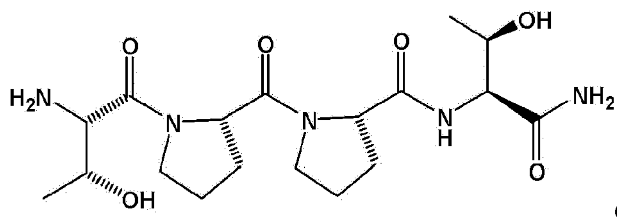

or salt thereof) in from about 2 mL to about 6 mL (e.g., from about 2.5 mL to about 5 mL, from about 2 mL to about 4 mL, from about 2.5 mL to about 3.5 mL, about 2.7 mL, about 3 mL, from about 4 mL to about 6 mL, from about 4.5 mL to about 5.5 mL, about 5mL) of an aqueous solution.

For example, the compositions described herein can have from about 350 mg to about 500 mg (e.g., from about 350 mg to about 450 mg, from about 400 mg to about 500 mg, about 400 mg, or about 450 mg) of the GLYX peptide (e.g., a GLYX-13 peptide and/or derivative and/or salt thereof; e.g., a GLYX peptide having the formula shown above or salt thereof) in from about 2 mL to about 4 mL, from about 2.5 mL to about 3.5 mL, about 2.7 mL, about 3 mL of an aqueous solution (e.g., about 400 mg of the peptide or salt in about 2.7 mL of an aqueous solution; or about 450 mg of the peptide or salt in about 3 mL of an aqueous solution). In some embodiments, the compositions have a pH of from about 4 to about 5, e.g., about 4.5, at 25 °C, and/or upon administration of the compositions, a desired physiological osmolality is achieved or maintained; see, e.g., the osmolality parameters described in paragraphs **[0032]** and **[0036]-[0038].**

As another example, the compositions described herein can have from about 600 mg to about 800 mg (e.g., from about 700 mg to about 800 mg, about 750 mg) of the GLYX peptide (e.g., a GLYX-13 peptide and/or derivative and/or salt thereof; e.g., a GLYX peptide having the formula shown above or salt thereof) in from about 4 mL to about 6 mL, from about 4.5 mL to about 5.5 mL, about 5mL) of an aqueous solution (e.g., about 750 mg of the peptide or salt in about 5 mL of an aqueous solution). In some embodiments, the compositions have a pH of from about 4 to about 5, e.g., about 4.5, at 25 °C, and/or upon administration of the compositions, a desired physiological osmolality is achieved or maintained; see, e.g., the osmolality parameters described in paragraphs **[0032]** and **[0036]-[0038].**

In some embodiments, the compositions described herein can be stored in a syringe (e.g., a 5 mL glass or plastic syringe). Contemplated herein, for example, is a syringe that includes a disclosed compound.

In some embodiments, the concentration of the GLYX peptide (e.g., a GLYX-13 peptide and/or derivative and/or salt thereof; e.g., a GLYX peptide having the formula shown above or salt thereof) that is utilized in the compositions described herein may be, for example, depend upon the physiological osmolality that is obtained upon administration. For example, at lower concentrations, e.g. below about 100 mg/mL, or below about 90 mg/mL or about 70mg/mL of the GLYX peptide, the compositions may be hypotonic (lower than physiological osmolality). Hypotonic compositions may lead to adverse patient reactions upon administration, e.g. an injectable hyportonic solution may cause blood cells to expand and break, also known as hemolysis. At higher concentrations, e.g., greater than 200 mg/mL of the GLYX peptide, the compositions (e.g., aqueous solutions) may be hypertonic. Hypertonic compositions may lead to adverse patient reactions upon administration, for example, causing blood cells to shrivel and become crenated.

In some embodiments, methods of administering and/or treating a disorder (e.g., depression) are provided, where upon administration of the compositions described herein, a physiological osmolality of from about 200 mOsmol/kg to about 1000 mOsmol/kg (e.g., from about 200 mOsmol/kg to about 500 mOsmol/kg, from about 500 mOsmol/kg to about 1,000 mOsmol/kg, from about 600 mOsmol/kg to about 950 mOsmol/kg,from about 800 mOsmol/kg to about 1,000 mOsmol/kg, from about 850 mOsmol/kg to about 950 mOsmol/kg or about 800 mOsmol/kg to about 900 mOsmol/kg) is maintained or achieved. In some of these embodiments, the duration of administration is less than about 8 hours. In certain embodiments, disclosed methods contemplate duration of injection times of from about 1 minute to about 5 minutes per dose of composition. In still other embodiments, disclosed methods contemplate duration of injection times of less than 1 minute (e.g., from about 1 second to about 55 seconds, from about 5 seconds to about 55 seconds,from about 5 seconds to about 45 seconds, from about 5 seconds to about 30 seconds, from about 5 second to about 15 seconds).

Disclosed herein, for example, are compositions that have a GLYX peptide (e.g., a GLYX-13 peptide and/or derivative and/or salt thereof; e.g., a GLYX peptide having the formula shown above or salt thereof) concentration of from about 60 mg/mL to about 200mg/mL, or about 100 mg/mL to about 200 mg/mL (e.g., about 150 mg/mL or about 75 mg/mL). Contemplated methods of treatment include administerating such compositions so upon administration of e.g., about 150 mg/ml of the GLYX-13, a physiological osmolality of about 800 to 1100 mOsmol/kg (or about 820 to 880 mOsmol/kg), or upon administration of e.g., about 75 mg/ml of the GLYX-13 about 375 to 475 mOsmol/kg is obtained.

In other embodiments, disclosed compositions may include an organic acid or other acid, such as one or more of fumaric acid, malic acid, lactic acid, hydrochloric acid, hydrobromic acid, acetic acid, citric acid, phosphoric acid, nitric acid, sulfuric acid, ascorbic acid, formic acid, propionic acid, butryic acid, valeric acid, caproic acid, oxalic acid, benzoic acid, carbonic acid, phenol, uric acid, p-toluenesulfonic acid, trifluromethanesulfonic acid, or carboxylic acid (wherein for example, a disclosed composition includes hydrochloric acid), wherein the composition has a pH of from about 4 to about 7 (e.g., from about 3.5 to about 6.5, from about 3.5 to about 5.5, from about 4 to about 6, from about 3.9 to about 5.5, from about 4.0 to about 5.0, from about 4.2 to about 5.0, from about 4.2 to about 4.8, about 4.0, about 4.5) at 25°C. For example, a disclosed composition may include an aqueous solution of GLYX-13 wherein the composition has been adjusted to about pH 4.5 (or e.g., 4.1 to about 5.5 at 25 °C) by an acid prepared at 5N, e.g., 5N HCl. Contemplated methods of treatment include administerating such compositions so upon administration a physiological osmolality of about 480 to about 960 mOsmol/kg is obtained. For example, contemplated herein are compositions having a GLYX-13 concentration of 120 to 150 mg/mL with osmolalities upon administration, (with a administration duration of e.g. less than 8 hours, less than 4 hours, e.g. 1 minute to about 8 hours, or about 1 to 5 minutes per dose) of about 290 to 360 mOsmol/kg.

Also contemplated herein are compositions that include e..g., a buffer (for example, an acid, for example hydrochloric acid, or chloride ions) and have a pH of about 4 to about 7 (e.g., from about 3.5 to about 6.5, from about 3.5 to about 5.5, from about 4 to about 6, from about 3.9 to about 5.5, from about 4.0 to about 5.0, from about 4.2 to about 5.0, from about 4.2 to about 4.8, about 4.0, about 4.5) at 25°C. Methods of treatment are provided that comprise administering such a composition to a patient by injection, such that the injection/administration time is from about 5 seconds to about 5 minutes (e.g., from about 5 seconds to about 2 minutes, from about 5 seconds to about 1 minute, from about 5 seconds to about 55 seconds, from about 5 seconds to about 45 seconds, from about 5 seconds to about 30 seconds, from about 5 seconds to about 15 seconds) and results in a physiological osmolality to between about 580 to about 720 mOsmol/kg.

Disclosed herein, for example, are compositions that have a GLYX peptide (e.g., a GLYX-13 peptide and/or derivative and/or salt thereof; e.g., a GLYX peptide having the formula shown above in e.g., paragraph [0027] or salt thereof) concentration of from about 100 mg/mL to about 200 mg/mL (e.g., about 150 mg/mL). Contemplated methods of treatment include administerating such compositions so upon administration a physiological osmolality of from about 500 mOsmol/kg to about 1,000 mOsmol/kg (e.g., from about 600 mOsmol/kg to about 950 mOsmol/kg, from about 800 mOsmol/kg to about 1,000 mOsmol/kg, from about 850 mOsmol/kg to about 950 mOsmol/kg) is obtained.

In some embodiments, the compositions described herein include an acid, for example hydrochloric acid, and have a pH of about 4 to about 7 (e.g., from about 3.5 to about 6.5, from about 3.5 to about 5.5, from about 4 to about 6, from about 3.9 to about 5.5, from about 4.0 to about 5.0, from about 4.2 to about 5.0, from about 4.2 to about 4.8, about 4.0, about 4.5) at 25°C. Contemplated methods of treatment include administerating such compositions so upon administration a physiological osmolality of from about 500 mOsmol/kg to about 1,000 mOsmol/kg (e.g., from about 600 mOsmol/kg to about 950 mOsmol/kg, from about 800 mOsmol/kg to about 1,000 mOsmol/kg, from about 850 mOsmol/kg to about 950 mOsmol/kg) is obtained. For example, the compositions described herein can have a GLYX peptide (e.g., a GLYX-13 peptide and/or derivative and/or salt thereof; e.g., a GLYX peptide having the formula shown above in e.g., paragraph **[0027]** or salt thereof) concentration of from about 125 mg/mL to about 175 mg/mL (e.g., about 150 mg/mL) with osmolalities upon administration of from about 600 mOsmol/kg to about 950 mOsmol/kg (e.g., from about 800 mOsmol/kg to about 1,000 mOsmol/kg, from about 850 mOsmol/kg to about 950 mOsmol/kg). In certain of these embodiments, the compositions are administered with an administration duration of, e.g., less than 8 hours, less than 4 hours; e.g., from less than one minute to 8 hours, 4 hours, 2 hours, 1 hour, or 0.5 hours; from about 1 minute to about 8 hours, 4 hours, 2 hours, 1 hour, or 0.5 hours; from about 1 minute to about 5 minutes, less than one minute, e.g., from about 1 second to about 55 seconds, from about 5 seconds to about 55 seconds,from about 5 seconds to about 45 seconds, from about 5 seconds to about 30 seconds, from about 5 second to about 15 seconds) per dose.

In some embodiments, the compositions described herein include, e..g., a buffer (for example, an acid, for example hydrochloric acid, or chloride ions) and have a pH of about 3.5 to about 6 (e.g., from about 3.9 to about 5.5, from about 4.0 to about 5.0, from about 4.2 to about 5.0, from about 4.2 to about 4.8, about 4.0, about 4.5) at 25°C. Methods of treatment are provided that comprise administering such a composition to a patient by injection, such that the injection/administration time is from about 5 seconds to about 5 minutes (e.g., from about 5 seconds to about 2 minutes, from about 5 seconds to about 1 minute, less than one minute, e.g., from about 1 second to about 55 seconds, from about 5 seconds to about 55 seconds,from about 5 seconds to about 45 seconds, from about 5 seconds to about 30 seconds, from about 5 second to about 15 seconds)) and result in a physiological osmolality of from about 600 mOsmol/kg to about 950 mOsmol/kg (e.g., from about 800 mOsmol/kg to about 1,000 mOsmol/kg, from about 850 mOsmol/kg to about 950 mOsmol/kg).

Disclosed aqueous compositions are stable in that the compound does not rapidly degrade or break down while in solution over time. Thus, in certain embodiments, a disclosed composition has minimal amounts of one or more of degradation products each selected from the group consisting of cyclo-Pro-Thr (proline-threonine-diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days or more, after 30 days or more, after 40 days or more, or after 60 days or 90 days or more at room temperature. In certain other embodiments, a disclosed composition has minimal amounts of one or more of degradation products each selected from the group consisting of cyclo-Pro-Thr , Thr-Pro-Pro-Thr, Pro-Pro-Thr, Pro-Pro-Thr-$NH_2$, Thr-Pro, Pro-Thr, Pro-Thr-$NH_2$, proline and/or threonine after 20 days at room temperature. For example, disclosed compositions may have minimal amounts of the compound:

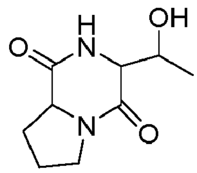

In certain other embodiments, a disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 3 month at from about 0°C to 10 °C (e.g., from about 2°C to 8 °C, about 5 °C), 6 months at from about 0°C to 10 °C (e.g., from about 2°C to 8 °C, about 5 °C), after 9 months at from about 0°C to 10 °C (e.g., from about 2°C to 8 °C, about 5 °C), after 12 months at from about 0°C to 10 °C (e.g., from about 2°C to 8 °C, about 5 °C), after 5 months at from about 0°C to 10 °C (e.g., from about 2°C to 8 °C, about 5 °C), or even after 24 months or more at from about 0°C to 10 °C (e.g., from about 2°C to 8 °C, about 5 °C).

In certain embodiments, the buffer is selected from the group consisting of acetate, citrate, phosphate, succinic acid, carbonate, bicarbonate, and maleic acid and salts thereof. In another embodiment, the buffer is a salt selected from the group consisting of chloride, sodium, potassium, and ammonium. In yet another embodiment, the GLYX peptide (e.g., a GLYX-13 peptide and/or derivative and/or salt thereof; e.g., a GLYX peptide having the following formula:

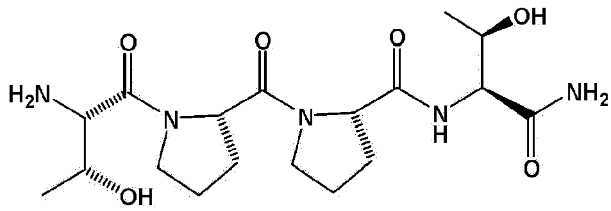

or salt thereof) may act as the buffer itself (with, e.g., a pKa of about 7 to about 7.5). In these embodiments, the compositions described herein comprise the GLYX peptide (e.g., a GLYX-13 peptide and/or derivative and/or salt thereof; e.g., a GLYX peptide having the formula shown above or salt thereof) and an acid (e.g., to obtain a desired pH) selected from the group consisting of hydrochloric acid, phosphoric acid, or sulfuric acid (e.g., hydrochloric acid).

In another embodiment, an aqueous composition is provided that consists essentially of water, the GLYX peptide (e.g., a GLYX-13 peptide and/or derivative and/or salt thereof; e.g., a GLYX peptide having the formula shown above or salt thereof) and an acid. In some embodiments, the compositions include a cationic counterion that is selected from the group consisting of $H^+$, a protonated form of the peptide, one of more protonated froms of any one or more of the degradation products described herein, or a combination thereof. In certain embodiments, the cationic counterion is selected from the group consisting of $H^+$ and a protonated form of the peptide, or a combination thereof. In certain embodiments, the compositions are substantially free of cationic sources other than those delineated above, e.g., metal cations, exogenous protonated amino acids or peptides, tetraalkyl ammonium ions, and other protonated acid scavengers.

In certain embodiments, the compositions are substantially free of anions other than chloride, e.g., are substantially free of acetate and other carboxylate-containing moieties, bromide, iodide, sulfate-containing moieties, sulfinate-containing moieties, and phosphate-containing moieties.

In certain embodiments, a cosolvent is present in a disclosed composition and is selected from the group consisting of polyethylene glycol, glycerine, ethanol, polypropylene glycol, and N,N-diemethylacetamide. For example, in certain embodiments, the cosolvent is polyethylene glycol having a molecular weight of about 200 to about 900Da. In certain embodiments, the cosolvent is polyethylene glycol having a molecular weight of about 400Da, e.g. about 400Da to about 700Da, .e.g., 200, 300, 400, 500, 500, 700 or 800 Da.

In certain embodiments, a disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 week, 10 days, 2 weeks, 3 weeks, 4 weeks, 2 months, 3 months, 4 months, 5 months, 6 months, 9 months, 1 year, 2 years, or more at from about 2 to about 8°C. In another embodiment, a disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 week, 10 days, 2 weeks, 3 weeks, 4 weeks, 2 months, 3 months, 4 months, 5 months, or 6 months, 12 months or more at 25°C.

In yet another embodiment, a disclosed composition has less than 0.5 weight percent (or, e.g, less than 0.7 weight percent, or less than 1 weight percent, less than 2 weight percent, less than 3 weight percent, less than 4 weight

percent, , e.g. about 1 to 3 weight percent) of Thr-Pro-Pro-Thr (des amide of GLYX-13) after 3 months, or after 6 months, or after 12 months or more. In another embodiment, the composition has less than 0.1 weight percent Thr-Pro-Pro-Thr after about 10 days, or after about 20 days, or after about 30 days.

In yet another embodiment, a disclosed composition has less than about 0.5 or less than about 0.6 weight percent (or, e.g, less than about 1.2 weight percent or less than about 0.7 weight percent, less than 1 weight percent, less than 2 weight percent, less than 3 weight percent, less than 4 weight percent, less than 5 weight percent, less than 6 weight percent, less than 7 weight percent, where the weight percent of the impurity is a percentage of the pharmaceutically active compound, e.g., GLYX-13) cyclo-Pro-Thr after 3 months, or after 6 months, or after 12 months, 24 months or more at 8°C. In another embodiment, a disclosed composition has less than about 0.5 weight percent cyclo-Pro-Thr after about 10 days, or after about 20 days, or after about 30 days at 40°C. In still another embodiment, the compositions can include up to about 5 weight percent, e.g. about 0.01 to about 5 weight percent, e.g. about 4 to about 5 weight percent diketopiperazine upon administration.

In yet another embodiment, a disclosed composition has less than about 0.5 weight percent (or, e.g, less than about 0.7 weight percent or less than about 1 weight percent) Pro-Thr-$NH_2$ after 10 days, or after 20 days, or after 30 days or more. In another embodiment, the composition has less than 0.3 weight percent Pro-Thr-$NH_2$ after about 10 days, or after about 20 days, or after about 30 days at 40°C.

In a certain embodiment, a disclosed composition has less than about 0.5 weight percent (or, e.g., less than about 0.1 weight percent, or less than 0.4 weight percent or less than 1 weight percent) Thr-Pro-Pro after 10 days, or after 20 days, or after 30 days. In another embodiment, the composition has less than 0.1 weight percent Thr-Pro-Pro after about 10 days, or after about 20 days, or after about 30 days at 40°C.

Disclosed compositions are stable in that the compound does not rapidly degrade or break down while in solution over time. In some embodiments, less than 5% (e.g., less than 4%, less than 3%, less than 2%, less than 1%, less than 0.5%) of the compound rapidly degrades or breaks down while in solution over time (e.g., 10 or more days) and at a temperature that is at, below, or above room temperature.

In certain embodiments, less than 5% (e.g., less than 4%, less than 3%, less than 2%, less than 1%, less than 0.5%) of the compound rapidly degrades or breaks down while in solution after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C, 40 °C, 45 °C; e.g., 40 °C). In an embodiment, less than 5% (e.g., less than 4%, less than 3%, less than 2%, less than 1%, less than 0.5%) of the compound rapidly degrades or breaks down while in solution after 21 days at 40°C.

In other embodiments, less than 5% (e.g., less than 4%, less than 3%, less than 2%, less than 1%, less than 0.5%) of the compound rapidly degrades or breaks down while in solution after 10 days or more, after 30 days or more, after 60 days or more, after 90 days or more, after 6 months or more, or after one year or more at room temperature.

In still other embodiments, less than 5% (e.g., less than 4%, less than 3%, less than 2%, less than 1%, less than 0.5%) of the compound rapidly degrades or breaks down while in solution after 1 month at 0 °C or below, after 3 months at 0 °C or below, after 6 months at 0 °C or below, after 9 months at 0 °C or below, after 12 months at 0 °C or below, after 18 months at 0 °C or below, or after 24 months or more at 0 °C or below.

In certain embodiments, the compositions described herein can exhibit any two or more of the stability features delineated above.

In certain embodiments, the compositions contain minimal amounts of one or both of degradation products diketopiperazine and proline-threonine amide. In other embodiments, the compositions have minimal amounts of one or more degradation products selected from the group consisting of diketopiperazine, proline-threonine amide, and any one or more of the other degradation products described herein.

In certain embodiments, the compositions have less than 1 weight percent (e.g, less than 0.7 weight percent, less than 0.5 weight percent, less than 0.4 weight percent, less than 0.3 weight percent) of diketopiperazine after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C, 40 °C, 45 °C; e.g., 40 °C). In an embodiment, the compositions have less than 0.7 weight percent, less than 0.4 weight percent, or less than 0.3 weight percent of diketopiperazine after 21 days in solution at 40 °C. In still another embodiment, the compositions can include up to about 5 weight percent of diketopiperazine upon administration. The weight precent values provided above are determined from the following equation: (weight impurity)/(weight of remaining compound) * 100.

In certain embodiments, the compositions have less than 1 weight percent (or, .e.g, less than 0.7 weight percent, less than 0.5 weight percent, less than 0.4 weight percent, less than 0.3 weight percent, less than 0.2 weight percent, or less than 0.1 weight percent) of proline-threonine amide after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C, 40 °C, 45 °C; e.g., 40 °C). In an embodiment, the compositions have less than 0.3 weight percent or less than 0.2 weight percent of proline-threonine

amide after 21 days in solution at 40 °C. The weight precent values provided above are determined from the following equation:

$$(\text{weight proline-threonine amide})/(\text{weight of remaining compound}) * 100.$$

In some embodiments, the compositions are prepared by processes that include (i) providing a first combination comprising the compound and water; and (ii) contacting the first combination with hydrochloric acid, or a source thereof, in an amount sufficient to achieve a pH of from about 3.5 to about 6.5.

Embodiments can include any one of the features described above as well as any combination of two or more of the features described above.

Methods

The present disclosure relates in part to the use of the disclosed GLYX-13 intravenous compositions for treatment of a variety of other neurological conditions are expected to be treated according to the methods of the disclosure. Exemplary conditions include, but are not limited to, a learning disorder, autistic disorder, attention-deficit hyperactivity disorder, anxiety, depression, migraine, Tourette's syndrome, phobia, post-traumatic stress disorder, dementia, AIDS dementia, Alzheimer's disease, Parkinson's disease, Huntington's disease, spasticity, myoclonus, muscle spasm, bipolar disorder, neuropathic pain, a substance abuse disorder, urinary incontinence, stroke, ischemia, epilepsy and schizophrenia.

Contemplated methods include a method of treating autism and/or an autism spectrum disorder in a patient need thereof, comprising administering an effective amount of a disclosed composition (e.g. a composition described above) to the patient. In an embodiment, a method for reducing the symptoms of autism in a patient in need thereof is contemplated, comprising administering an effective amount of a disclosed composition to the patient. For example, upon administration, the composition may decrease the incidence of one or more symptoms of autism such as eye contact avoidance, failure to socialize, attention deficit, poor mood, hyperactivity, abnormal sound sensitivity, inappropriate speech, disrupted sleep, and perseveration. Such decreased incidence may be measured relative to the incidence in the untreated individual or an untreated individual(s).

In some embodiments, patients suffering from autism also suffer from another medical condition, such as Fragile X syndrome, tuberous sclerosis, congenital rubella syndrome, and untreated phenylketonuria.

In another embodiment, methods of treating a disorder in a patient need thereof are contemplated, wherein the disorder is selected from group consisting of: epilepsy, AIDS and/or AIDS dementia, Parkinson's disease, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, and impulse control disorders that includes administering a disclosed compound, e.g. GLYX-13. Also contemplated herein is a method for treating cough, e.g. uncontrollable cough, comprising administering a GLYX-13 composition to a patient in need thereof.

For example, provided here are methods of treating benign Rolanic epilepsy, frontal lobe epilepsy, infantile spasms, juveline myoclonic epilepsy, Lennox-Gastaut syndrome, Landau-Kleffner syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease, and /or photosensitive epilepsy comprising administering an effective amount of a disclosed composition.

In an embodiment, contemplated herein are methods of treating attention deficit disorder, ADHD (attention deficit hyperactivity disorder), schizophrenia (for example, schizo-affective disorders, delusional disorders, e.g., paranoid type, hebephrenic, and/or catatonic type schizophrenia), bipolar disorder (include bipolar I disorder, bipolar II disorder, cyclothymia), borderline personality disorder, anxiety,(include social anxiety disorder, avoidant personality disorder), obsessive-compulsive disorder, amelioration of opiate, nicotine and/or ethanol addiction (e.g., method of treating such addiction or ameliorating the side effects of withdrawing from such addiction), spinal cord injury diabetic retinopathy, traumatic brain injury, frontal temporal dementia, post-traumatic stress syndrome and/or Huntington's chorea, in a patient in need thereof, that includes administering a disclosed composition. For example, patients suffering from schizophrenia, addiction (e.g. ethanol or opiate), autism (and autism spectrum disorders), Huntington's chorea, traumatic brain injury, spinal cord injury, post-traumatic stress syndrome and diabetic retinopathy may all be suffering from altered NMDA receptor expression or functions.

For example, provided herein is a method of treating schizophrenia, for example, the negative and cognitive symptoms of schizophrenia in a patient suffering therefrom, comprising administering a therapeutically effective amount of a disclosed composition.

Contemplated herein is a method of treating stroke and/or ischemia, e.g., ischemic stroke, brain ischemia,

transient ischemic attack, cardiac ischemia,and/or myocardial infarction, in a patient in need thereof, comprising administering a pharmaceutically effective amount of a disclosed composition.

Also provided herein is a method of modulating an autism target gene expression in a cell comprising contacting a cell with an effective amount of a disclosed composition. The autism gene expression may be for example, selected from ABAT, APOE, CHRNA4, GABRA5,GFAP, GRIN2A, PDYN, and PENK. In another embodiment, a method of modulating synaptic plasticity in a patient suffering from a synaptic plasticity related disorder is provided, comprising administering to the patient an effective amount of a disclosed composition.

In another embodiment, a method of treating Alzheimer's disease, or e.g., treatment of memory loss that e.g., accompanies early stage Alzheimer's disease, in a patient in need thereof is provided, comprising administering a disclosed composition. Also provided herein is a method of modulating an Alzheimer's amyloid protein (e.g., beta amyloid peptide, e.g. the isoform A 1-42), in-vitro or in-vivo (e.g. in a cell) comprising contacting the protein with an effective amount of a disclosed composition. For example, in some embodiments, a disclosed composition may block the ability of such amyloid protein to inhibit long-term potentiation in hippocampal slices as well as apoptotic neuronal cell death. In some embodiments, a disclosed composition may provide neuroprotective properties to a Alzheimer's patient in need thereof, for example, may provide a therapeutic effect on later stage Alzheimer's-associated neuronal cell death.

Also contemplated herein is the use of a disclosed GLYX-13 composition for treatment of clinically relevant antidepressant and anxiolytic and for treatment of depression and anxiety in general.

In certain embodiments, the present invention relates at least in part to the use of a disclosed GLYX-13 composition alone or in combination with one or more other antidepressant treatments, such as, tricyclic antidepressants, MAO-I's, SSRI's, and double and triple uptake inhibitors and/or anxiolytic drugs for manufacturing a medicament for treating depression, anxiety, and/or other related diseases including provide relief from depression, anxiety and preventing recurrence of depression and anxiety. Exemplary drugs that may be used in combination with a GLYX peptide include Anafranil, Adapin, Aventyl, Elavil, Norpramin, Pamelor, Pertofrane, Sinequan, Surmontil, Tofranil, Vivactil, Parnate, Nardil, Marplan, Celexa, Lexapro, Luvox, Paxil, Prozac, Zoloft, Wellbutrin, Effexor, Remeron, Cymbalta, Desyrel (trazodone), and Ludiomill. It will be appreciated that in some embodiments, administration of a disclosed GLYX-13 composition may act more quickly than a co-administered antidepressant treatment, and thus such co-administration (e.g., administration of GLYX-13 on an acute or immediate basis, while starting a regimen with another, slower acting anti-depressant at about the same time) may be particularly advantageous in the common situation where the second antidepressant is slower acting.

Also contemplated herein are methods of treating depression that include administering a disclosed composition in combination with (e.g. simultaneously or sequentially) other non-pharmacological treatments such as psychotherapy, electroconvulsive therapy, vagus nerve stimulation and/or transcranial magnetic stimulation.

A variety of depression conditions are expected to be treated according to this aspect of the disclosure without affecting behavior or motor coordination, and without inducing or promoting seizure activity. Exemplary depression conditions that are expected to be treated according to this aspect of the disclosure include, but are not limited to, major depressive disorder, dysthymic disorder, psychotic depression, postpartum depression, premenstrual syndrome, premenstrual dysphoric disorder, seasonal affective disorder (SAD), anxiety, mood disorder, depressions caused by chronic medical conditions such as cancer or chronic pain, chemotherapy, chronic stress, post-traumatic stress disorders, risk of suicide, and bipolar disorder (or manic depressive disorder). It should be understood that depression caused by bipolar disorder may be referred to as bipolar depression. In addition, patients suffering from any form of depression often experience anxiety. Various symptoms associated with anxiety include fear, panic, heart palpitations, shortness of breath, fatigue, nausea, and headaches among others. It is expected that the methods of the present condition can be used to treat anxiety or any of the symptoms thereof.

Also provided herein are methods of treating depression in treatment resistant patients or treating refractory depression, e.g., patients suffering from a depression disorder that does not, and/or has not, responded to adequate courses of at least one, or at least two, other antidepressant compounds or therapeutics. For example, provided herein is a method of treating depression in a treatment resistant patient, comprising a) optionally identifying the patient as treatment resistant and b) administering an effective dose of a disclosed GLYX-13 composition to said patient.

Provided herein, in an embodiment, are methods of acutely treating symptoms of depression in a patient in need thereof, comprising administering an effective amount of GLYX-13, for example, in a single unit dose e.g. intravenously or subcutaneously. Such methods may relieve the patient of at least one symptom of depression for about 2 weeks or less, 1 week or less, 1 day or less, or 1 hour or less (e.g. 15 minutes or less, half an hour or less), after said administration. In some embodiments, such methods may relieve the patient of at least one symptom of depression for about 1 day or more, 1 week or more, or 2 weeks or more after said administration. For example, provided herein is a method comprising administering an effective amount of GLYX-13 to a patient suffering from depression, wherein said patient is substantially relieved of at least one symptom of depression substantially earlier after the first administration of GLYX-13, as compared to the same patient administered a non-GLYX-13 antidepressant compound. One of skill in the art will appreciate that such methods of acute administration may be advantageous in a hospital or out-patient setting.

Symptoms of depression, and relief of same, may be ascertained by a physician or psychologist, e.g., by a mental state examination. Symptoms include thoughts of hopelessness, self-harm or suicide and/or an absence of positive thoughts or plans.

In some embodiments, the patient is a human, e.g. a human pediatric patient.

In some embodiments, contemplated methods relate to use of a disclosed compositions alone or in combination with one or more other agents for manufacturing a medicament for treating depression or another contemplated indication.

GLYX-13 may provide a high therapeutic index. For example, GLYX-13 may be therapeutically effective with an i.v. dose range of about 1 to about 10 mg/kg. In some embodiments, no ataxia occurs, at for example a dose of at 500 mg/kg, i.v.

In an embodiment, a disclosed method includes administering one dose, or one or more doses, of a disclosed composition. In some embodiments, a patient has substantial improvement after 12 hours, after 1 day, after 1 week, after 2 days, after 3 days, after 4 days, after 5 days, after 6 days, or even after 8 days of a one (single) dose administration.

A therapeutically effective amount of a disclosed composition required for use in therapy varies with the nature of the condition being treated, the length of treatment time desired, the age and the condition of the patient, and is ultimately determined by the attending physician. In general, however, dosage forms employed for adult human treatment typically are in the range of about 10 mg/ml to about 70 mg/ml, or 70 to 200 mg/ml. In some embodiments, disclosed dosage forms are capable of delivering about 0.5 to 2 grams per day of GLYX-13 to a patient. A number of factors may lead to the disclosed composition being administered over a wide range of dosages. When given in combination with other therapeutic agents, the dosage of the disclosed compositions may be given at relatively lower dosages. As a result, the dosage form of a disclosed composition may be from about 10 mg/ml to about 70 mg/ml, or for example, about 70 mg/mL to about 200 mg/ml. The dosage of a disclosed composition may be at any dosage including, but not limited to, about 10 mg/ml, 15 mg/ml, 20 mg/ml, 25 mg/ml, 30 mg/ml, 35 mg/ml, 40 mg/ml, 45 mg/ml, 50 mgl/ml, 55 mg/ml, 60 mg/ml, 65 mg/ml, or 70 mg/ml. More concentrated solutions, including 80 mgl/ml, 100 mg/ml, 125 mg/ml, 150 mg/ml, or 200 mg/ml are also disclosed as convenient dosage forms. The desired dose may be conveniently administered in a single dose, or as multiple doses administered at appropriate intervals, for example as two, three, four or more sub-doses per day. Pharmaceutical compositions of the disclosure suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required particle size in the case of dispersions, and by the use of surfactants.

The invention now being generally described, it will be more readily understood by reference to the following examples which are included merely for purposes of illustration of certain aspects and embodiments of the present invention, and are not intended to limit the invention in any way.

## EXAMPLE 1 GLYX-13, 60mg/mL in saline solution, pH 6.5 to 7.0

Prepare 90 g/L of sodium chloride solution in water for injection USP (WFI).

Preparation of 180 mg/ml GLYX-13 Solution

Add water for injection USP (WFI) to a compounding vessel, to an amount equivalent to 20% of the final QS weight and record the exact weight added. Begin mixing the solution and add calculated amount of Acetic Acid USP to the compounding vessel. The amount of acetic acid is 0.9 to 1.0 molar equivalent of the target amount of GLYX-13 in the formulation batch.

Add the calculated amount of GLYX-13 free base to the compounding vessel and record the exact weight added. Continue mixing for 15 to 30 minutes.

Stop mixing and add calculated amount of 90 g/L saline solution to the compounding vessel. The amount of NaCl must be equal to the amount needed reach 9 g/L in the final formulation.

Preparation of 60 mg/ml GLYX-13 Solution

Transfer the entire content of the compounding vessel into a polymeric mixing bag or large stainless steel mixing vessel. Mix for 15-30 minutes. Add sufficient amount of WFI to the solution to reach the final target final volume.

Test the content of GLYX-13 by HPLC and test the pH. If the pH is outside the range of 6.6 to 6.9, adjust the pH with acetic acid or NaOH. The final batch will contain, per liter of solution, 60 grams GLYX-13 and 6 to 6.4 grams Acetic Acid in 0.9 % sodium chloride; the pH must be between 6.5 and 7.0.

Sterilization and Vial Filling

The solution is sterilized by aseptic filtration through the following three filters in series: a pre-filtration with 0.45 μm filter, followed by two 0.22 μm filters. Fill each vial with 20ml (20 to 20.5 mL) of the bulk sterile solution, stopper and cap vials. Each vial contains 1.2 grams of GLYX-13 in 20 mL solution.

**EXAMPLE 2 GLYX-13 in Buffer Solutions**

A. Prepare 0.1 M Tris Buffer Solution ("TrisHCl-7.0") in water, pH 7.0 using HCl to adjust the pH.

Preparation of 60 mg/ml GLYX-13 Solution

Add approximately 75% the target volume of TrisHCl-7.0 buffer to a compounding vessel. For each liter of the target batch volume, weigh 60 g of GLYX-13 free base and add to the compounding vessel. Mix for 15 min. Measure the pH. If pH is out of range (7.0±0.05), adjust accordingly with HCl or NaOH. Add enough TrisHCl-7.0 to reach the final batch volume. The final bulk solution will contain, per liter of solution, 60 grams GLYX-13 free base in 0.1 M Tris(hydroxymethyl)aminomethane/HCl buffer, pH 7.0.

Sterilization and Vial Filling

Filter sterilize and fill the vials as described above. Each vial contains 1.2 grams of GLYX-13 in 20 mL solution (GLYX-13 60 mg/mL, tris(hydroxymethyl)aminomethane/HCl buffer, pH 7.0).

B. 60 mg/mL in Citrate Buffer solution, pH 5.6: Prepare 0.1 M trisodium citric acid solution in water and adjust the pH to 5.6 with HCl and NaOH as needed.

Preparation of 60 mg/ml GLYX-13 Solution

Add approximately 75% the target batch volume of citric acid/sodium citrate buffer, pH 5.6 to a compounding vessel. For each liter of the target batch volume, weigh 60 g of GLYX-13 free base and add to the compounding vessel. Mix for 15 min. Measure the pH. If pH is out of range (5.6±0.05), adjust accordingly with HCl or NaOH. Add enough citric acid/sodium citrate buffer (pH 5.6) to reach the final batch volume. The final bulk solution will contain, per liter of solution, 60 grams GLYX-13 free base in 0.1 M citric acid/sodium citrate buffer, pH 5.6.

C. Sterilization and Vial Filling

Filter sterilize and fill the vials as described above. Each vial contains 1.2 grams of GLYX-13 in 20 mL solution. (GLYX-13 in citric acid/sodium citrate buffer, pH 5.6)

C. GLYX-13, 200 mg/mL in Tris Buffer solution, pH 7.5

A. Prepare 0.1 M Tris Buffer Solution ("TrisHCl-7.5") in water, pH 7.5 using HCl to adjust the pH.

B. Preparation of 200 mg/ml GLYX-13 Solution

Add approximately 50% the target volume of TrisHCl-7.5 buffer to a compounding vessel. For each liter of the target batch volume, weigh 200 g of GLYX-13 free base and add to the compounding vessel. Mix for 15 min. Measure the pH. If pH is out of range (7.5±0.05), adjust accordingly with HCl or NaOH. Add enough TrisHCl-7.5 to reach the final batch volume. The final bulk solution will contain, per liter of solution, 200 grams GLYX-13 free base in 0.1 M Tris(hydroxymethyl)aminomethane/HCl buffer, pH 7.5.

Sterilization and Vial Filling

Filter sterilize and fill the vials as described above. Fill each vial with 5 ml (4.85 to 5.15 mL) of the bulk sterile solution, stopper and cap vials. Each vial contains 1 grams of GLYX-13 in 10 mL solution. (GLYX-13 200 mg/mL, Tris(hydroxymethyl)aminomethane/HCl buffer, pH 7.5)

D. GLYX-13, 200 mg/mL in Tris Buffer and 40% PEG400 solution, pH 7.5

. Prepare 0.1 M Tris Buffer Solution ("TrisHCl-7.5") in water, pH 7.5 using HCl to adjust the pH.

Preparation of 200 mg/ml GLYX-13 Solution

Add approximately 20 L of 0.2 M TrisHCl-7.5 buffer to a compounding vessel. Add 40 L of PEG400 to the vessel and stir for 30 min. Weigh 20 kg of GLYX-13 free base and add to the compounding vessel. Mix for 0.5 to 2 hours. Remove a sample and measure the pH. If pH is out of range (7.5±0.05), adjust accordingly with HCl or NaOH. Add enough TrisHCl-7.5 (approximately 30 L) to reach the final batch volume of 100 L. The final bulk solution will contain, per liter of solution, 200 grams GLYX-13 free base and 40% PEG400 in 0.1 M Tris(hydroxymethyl)aminomethane/HCl buffer, pH 7.5.

Sterilization and Vial Filling

Filter sterilize and fill the vials as described above. Fill each vial with 10 ml (10 to 10.5 mL) of the bulk sterile solution, stopper and cap vials. Each vial contains 2 grams of GLYX-13 in 10 mL solution. (GLYX-13 200 mg/mL, in 40 % Polyethylene glycol 400 (PEG400), Tris(hydroxymethyl)aminomethane/HCl buffer, pH 7.5)

**EXAMPLE 3 Stability of GLYX-13 I.V. Solutions**

The stability of GLYX-13 is tested by HPLC using the conditions below.

HPLC Analytical Method

Column: RP-C18 ODS 2, (5 μm, 80A, 4.6 x 250 mm)
Eluent A: 13.4 g potassium dihydrogen phosphate and 4.4 g 1-heptanesulfonic acid sodium salt in 1600 mL water. Adjust pH to 2.5 with o-phosphoric acid. Dilute to 2 L with water.
Eluent B: 1400 mL Methanol and 600 mL Water.
Gradient: 20% B to 70% B in 25 min.; 70% B to 20% B in 3 min.; 0% B for 8 min.
Flow Rate: 1 mL/min.
Detection: UV (220 nm)
Injection: 20 μL of 1 mg/mL solution
Temperature: Ambient

The retention time for GLYX-13 is 12.0 to 12.8 minutes. Thr-Pro-Pro-Thr (TPPT = deaminated GLYX-13) has a retention time of 14.9 to 15.1 minutes. Thr-Pro-Pro (TPP) has retention time of 4.8 to 5.2 minutes. Impurity peaks using this method are analyzed in terms of percent (%) area, versus the GLYX-13 peak and not presented by weight/weight percentage.

**EXAMPLE 4 Low pH Formulations**

GLYX-13 was formulated at 150 mg/mL and adjusted to specific pH with either acetic acid or hydrochloric acid. The formulations were then subjected to accelerated stability conditions (2 months at 40° C). Table 1 provides a summary of the key stability-limiting attributes versus pH and counter ions (acetic acid and HCl).

**Table 1. Stability of GLYX-13 Solutions at 40° C for 2 months.**

| pH<br>Counter ion | AA-5<br>5.0<br>Acetic Acid | HCl-5<br>5.0<br>HCl | HCl-4<br>4.0<br>HCl | HCl-8<br>8.0<br>HCl |
|---|---|---|---|---|
| Assay: % Label Claim | 62.0 | 89.3 | 90.8 | 76.5 |

(continued)

| pH<br>Counter ion | AA-5<br>5.0<br>Acetic Acid | HCl-5<br>5.0<br>HCl | HCl-4<br>4.0<br>HCl | HCl-8<br>8.0<br>HCl |
|---|---|---|---|---|
| Impurity 1. RRT 0.43 (% area) | 16.0 | 0.97 | 0.69 | 1.87 |
| Impurity 2. RRT 0.57 (% area) | 4.4 | 0.34 | 0.25 | 0.54 |
| Total Impurities[1] (% area) | 22.37 | 2.54 | 2.22 | 13.50 |
| 1.At the beginning of the study, the % claim was 93%, and the total impurity levels were 1.6%. Impurities are reported as % of the GLYX-13 peak from the sample. Total impurities includes total of all individual impurities at levels greater than 0.05%. Impurities 1 and 2 represent the two major impurities that increase over time. The rate of impurity formation is dependent on counter ion, pH, and temperature. | | | | |

Table 1 indicates that HCl as the counter ion stabilizes the formulation relative to acetic acid. At pH 5.0 the levels of impurities are 10-fold lower and the % label claim (150 mg) of GLYX-13 is almost 50% higher in HCl solution versus acetic acid solution (see Forms AA-5 versus HCl-5). Further, lower pH solutions are more stable than high-pH solutions: note that in HCl solutions, the levels of impurities are significantly lower and the % label claim (150 mg) of GLYX-13 are significantly higher at low pH (4 and 5) versus higher pH (8) solution (see Formulations HCl-4 and HCl-5 versus Form HCl-8).

**EXAMPLE 5 Syringe/Container Stability Study**

The objective of this study was to examine the stability of the GLYX-13 150 mg/mL formulation filled into syringes and vials. Four different syringe and stopper combinations were examined along with one vial and stopper configuration.

**Table 2**

| Container | Closure |
|---|---|
| BD glass syringes | HyPak SCF plunger |
| BD glass syringes | Sterifill plunger |
| Schott TopPac syringes | FM257 Plunger |
| Schott TopPac syringes | Stelmi Plunger |
| Schott glass vial | 13 mm stopper |

A Flexicon peristaltic pump equipped with Flexicon tubing was calibrated to deliver specific fill volumes based on the density of the drug solution. The density of the drug solution at 25°C was 1.044 g/mL. The glass syringes were filled with 5.1 mL or 5.32 g of solution and the polymeric syringes were filled with 5.14 mL or 5.37 g of solution. Extra solution was included in each syringe to account for volume retained by the syringe / stopper components that is unavailable for delivery. Each vial was filled with 5.32 g of solution to account for the volume retained by the vial.

The stability of the drug solution was compared with a placebo solution filled into the same container/closure combinations. Samples were stored at -20°C, 2-8°C, 25°C, and 40°C for the durations listed in the Tables 3 - 6.

Testing Key for the tables:

Samples containing active at 150 mg/mL:

A = pH, appearance, assay / related substances, color, deliverable volume (1 syringe required for all testing per time point.)

B = Deliverable volume, HIAC, osmolality, and optical rotation (5 syringes needed per time point.)

Samples containing placebo:

A = pH, appearance, assay / related substances, color (1 syringe required per time point.) B = Deliverable volume, HIAC (5 syringes required per time point.)

**Table 3:** Time Points and Testing for Samples Stored at -20 °C

| Time Points | Testing |
|---|---|
| T0 | A, B |
| 12 weeks | A, B |

**Table 4: Time Points and Testing for Samples Stored at 2-8 °C**

| Time Points | Testing |
|---|---|
| T0 | A, B |
| 4 weeks | A |
| 8 weeks | A, B |
| 12 weeks | A, B |
| 24 weeks | A, B |

**Table 5: Time Points and Testing for Samples Stored at 25 °C**

| Time Points | Testing |
|---|---|
| T0 | A, B |
| 4 weeks | A |
| 8 weeks | A, B |
| 12 weeks | A, B |
| 24 weeks | A, B |

**Table 6: Time Points and Testing for Samples Stored at 40 °C**

| Time Points | Testing |
|---|---|
| T0 | A, B |
| 2 weeks | A |
| 4 weeks | A |
| 6 weeks | A |
| 8 weeks | A, B |
| 12 weeks | A, B |

The testing and specifications for the syringes a Samples are tested for the following a selected time points. Not all tests are performed at each timepoint.: pH, appearance, assay / related substances, color, deliverable volume, particle size distribution (HIAC), osmolality, and optical rotation.

**Table 3:** Time Points and Testing for Samples Stored at -20 °C

| Time Points |
|---|
| Initial (T0) |

(continued)

| Time Points |
|---|
| 12 weeks |

**Table 4: Time Points and Testing for Samples Stored at 2-8 °C**

| Time Points |
|---|
| Initial (T0) |
| 4 weeks |
| 8 weeks |
| 12 weeks |
| 24 weeks |

**Table 5: Time Points and Testing for Samples Stored at 25 °C**

| Time Points |
|---|
| Initial (T0) |
| 4 weeks |
| 8 weeks |
| 12 weeks |
| 24 weeks |

**Table 6: Time Points and Testing for Samples Stored at 40 °C**

| Time Points |
|---|
| Initial (T0) |
| 2 weeks |
| 4 weeks |
| 6 weeks |
| 8 weeks |
| 12 weeks |

The testing and speficiations for the syringes and vials in the stability study are listed in Table 7. Testing was performed initially and after 4, 8, 12, and 24 weeks. Samples from the 2 week time point were frozen and tested at the same time as the 4 week samples. The samples from the 6 week time point were frozen and tested at the same time as the 8 week samples.

The syringes were stored in bags so that they remained horizontal during storage. Approximately 65 vials were stored in the upright position and 55 vials were stored inverted. The inverted samples were removed after 4, 8, 12, and 24 weeks of storage at each condition. Extra syringes were included per time point.

**Table 7: Testing and Specifications for the Drug Solution**

| Attribute | Test Method | Acceptance Criteria |
|---|---|---|
| Appearance | TBD | Clear solution, colorless to faint yellow, essentially free of visible contamination |
| UV Absorbance at TBD $cm^{-1}$ | TBD | Report Result |

(continued)

| Attribute | Test Method | Acceptance Criteria |
|---|---|---|
| Optical rotation | TBD | Report Result |
| Osmolality | USP<785> | Report Result |
| Identification | HPLC | Retention time of the sample corresponds to the retention time of the standard |
| Assay | HPLC | 90.0-110.0 %LC |
| Chromatographic Purity, Related Substances (%Area) | HPLC | (report any individual ≥0.05%)<br>Individual unspecified impurities: ≤0.5%<br>Total Unspecified: NMT 2.0%<br>Specified Unidentified impurities:<br>NRX-2181 (Thr-Pro-Pro-Thr), NMT 0.5%<br>NRX-1160 (Cyclo-Pro-Thr), NMT 2.0%.<br>NRX-1152 (Pro-Thr-NH2), NMT 1.0%.<br>NRX-1161 (Pro-Thr), NMT 2.0%.<br>Total impurities: ≤5.0% |
| pH | USP <791> | GLYX-13 4.5 - 5.0 |
| Particulate Matter | USP <788> small volume injections Method 2 | 2:10µm NMT 6000 particles<br>≥25µm NMT 600 particles |
| Volume per Container | USP <1> | Not less than 5 mL |

There was no significant difference in syringe container/closure configurations. All container/closure configurations met the acceptance criteria for appearance, identity, assay, specified related substances, total related substances, pH (active), particulate matter (active), and volume per container. Individual unspecified related substances and total unspecified related substances after 4 and 8 weeks for all container/closure configurations did not meet acceptance criteria in some instances.

**EXAMPLE 6 Stability Study**

Test and Control Articles

GLYX-13 Drug substance; and 5 M hydrochloric acid solutions; Methanol; 10 mL vial with 20 mm opening; 20 mm Daikyo solution stopper; Syringe filters, 0.2µm Millex-GV HPLC Method

Column: Waters Spherisorb ODS2, 3 µm, 4.6 x 250 mm, Part # PSS832115

Mobile phase A: 2.2 g/L heptanesulfonic acid, 6.7 g/L potassium dihydrogen phosphate, pH 2.5

Mobile phase B: 70/30 Methanol/Water

Needle wash: water

Column temperature: 40 ± 1 °C

Sample temperature: 4 ± 1 °C

Wavelength: 210 nm

Run time: 50 min

Flow rate: 1.0 mL/min

Gradient:

| Time | %B |
|---|---|
| 0 | 0 |
| 35 | 50 |
| 40 | 70 |
| 41 | 00 |
| 50 | |

Study

Four formulations were prepared to examine dissolution of the drug based (Table 2). Each formulation was prepared at 35 mL and contained 5.25 g of GLYX-13.

**Table 8. Formulations for the pH Study**

| Formulation | GLYX-13 (mg/mL) | pH | pH Adjustment Acid |
|---|---|---|---|
| 1 | 150 | 4 | HCl |
| 2 | 150 | 5 | HCl |
| 3 | 150 | 6 | HCl |
| 4 | 150 | 7 | HCl |

Each formulation was prepared by adding the drug to a beaker and adjusting the volume to approximately 26 mL using purified water. The pH was adjusted using 5 M HCl that was added in small increments. The solution was mixed for 10 minutes after each adjustment and mixed for 30 minutes after reaching the desired pH. The pH of the solution was checked after 30 minutes and adjusted if needed. The solution was adjusted to 35 mL using purified water

when a drift in pH was no longer observed. Each formulation was filled into vials (2.5 mL/vial), sealed with a stopper, and capped. The formulations were stored at 40°C for 3 weeks and samples were removed weekly. All weekly samples were examined for appearance, tested for pH, assay and related substances using the HPLC assay.

**Table 9. Stability of GLYX-13 Solutions at 40° C for 3 weeks.**

| **Form Code**<br>**pH**<br>**Counter ion** | **HCL-4**<br>**4.0**<br>**HCl** | **HCL-5**<br>**5.0**<br>**HCl** | **HCL-6**<br>**6.0**<br>**HCl** | **HCL-7**<br>**7.0**<br>**HCl** |
|---|---|---|---|---|
| Assay: % Label Claim | 102 | 102 | 101 | 96 |
| Impurity 1. RRT 0.43 (%) | 0.29 | 0.34 | 0.63 | 1.87 |
| Impurity 2. RRT 0.57 (%) | 0.13 | 0.16 | 0.25 | 0.68 |
| Total Impurities[1] (%) | 1.62 | 1.67 | 2.09 | 4.57 |
| 1. At the beginning of the study, the % label claim was 104%, and the total impurity levels were 1.2%. Impurities are reported as % area of the GLYX-13 peak from the sample. Total impurities includes total of all individual impurities at levels greater than 0.05%. Impurities 1 and 2 represent the two major impurities that increase over time. | | | | |

Data were analyzed using the appropriate software designed for the assay and impurities HPLC method. Table 3 provides a summary of the stability data at the 3-weeks testing point. Impurity 1 (RRT 0.43) is cyclo-proline-threonine ("diketopiperazine"). Impurity 2 (RRT 0.57) is proline-threonine amide. Figure 1 shows the formation of impurity 1 (RRT 0.43) over time versus pH. Figure 2 shows the formation of Impurity 2 (RRT 0.57) over time versus pH. All data is reported as % area of the GLYX-13 peak - not on weight/weight%).

The above examples indicate that the solutions maintained at low pH and neutral pH are more stable than those maintained at high pH. For example, the % (150 mg) of GLYX-13 is significantly higher at low pH (4 and 5) versus higher pH (7) solution (see Forms HCl-4 and HCl-5 versus Form HCl-7); the levels of the two specified impurities increase significantly with increasing of pH and the level of total impurities of GLYX-13 solutions at low pH stored at 40° C for 3 weeks is 3-fold lower at pH 4 relative to pH 7 solution.

The results show that the stability of GLYX-13 solution is inversely proportional to the pH. The rate of impurity formation at pH 7 is approximately 6-fold greater than the rate at pH 4. Such improved stability of a peptide in HCl at low pH e.g. about 4 to about 5, relative to neutral pH (6-8, e.g. 7) is surprising, in part because peptides are typically subject to hydrolysis at low pH in HCl. In addition, the decrease in GLYX-13 concentration/increase in GLYX-13 degradation products observed during stability testing were less for formulations prepared with HCl than with those prepared with acetic acid.

Example 6 Acid Study

The purpose of this study is to test the effect of the type of acid used for pH adjustment on the stability of rapastinel (GLYX-13) 150 mg/ml.

Rapastinel 150 mg/ml solution is prepared with rapastinel powder dissolved in water and the pH is adjusted to 4.5 using 5N HCl. This protocol describes the preparation of rapastinel 150 mg/ml solution with a pH adjust to 4.5 using 10 different types of acid all prepared at 5N - except as noted below.

Table 10. Materials, Suppliers for formulations for testing.

| **Material** | **Supplier** |
|---|---|
| Purified Water | Milli-Q |
| Fumaric Acid | Acros |
| Malic Acid | Acros |
| Lactic Acid | Sigma Aldrich |
| 5N HCl | Baxter |
| Acetic Acid | EMD |
| Citric Acid | Sigma Aldrich |

(continued)

| Material | Supplier |
|---|---|
| Phosphoric Acid | EMD |
| Nitric Acid | JT Baker |
| Sulfuric Acid | Sigma |
| Ascorbic Acid | JT Baker |

Table 11. Equipment List.

| Equipment | Supplier |
|---|---|
| Orion pH Meter Model 920A | Thermo Scientific |
| Vacuum Filtration System 0.2 micrometer PES PES Filter | VWR |

I. Acid Preparation

A. 5N HCl Solution This study will use 5N HCl solution prepared previously.

B. Fumaric Acid Solution

Molecular weight of fumaric acid = 116.07 x 5N = 580.35 mg/mL x 250 mL =145087.5 mg. Fumaric acid has low solubility in water, so the solution is prepared near its maximum solubility and concentrated solutions of rapastinel (450 mg/mL) is used to facilitate pH adjustment.

C. Malic Acid Solution

Molecular weight of malic acid = 134.09 x 5N = 670.45 mg/mL x 250 mL =167612.5 mg Molecular weight of malic acid = 116.07 x 5N = 580.35 mg/mL x 250 mL =145087.5 mg. Malic acid has low solubility in water, so the solution is prepared near its maximum solubility and concentrated solutions of rapastinel (450 mg/mL) is used to facilitate pH adjustment.

D. Lactic Acid Solution

The solution is prepared near its maximum solubility and concentrated solutions of rapastinel (450 mg/mL) is used to facilitate pH adjustment.

E. 5N Acetic Acid Solution (250 mL)

Molecular weight of acetic acid= 60.05 x 5N = 300.25 x 250 mL = 75062.5 mg

1. Add 75.06 g acetic acid to a 250 ml volumetric flask.
2. QS to 250 mL using purified water and mix thoroughly.

F. 5N Citric Acid Solution (250 mL)

Molecular weight of citric acid = 192.12 x 5N = 960.6 x 250 mL = 240150 mg

1. Add 240.15 g citric acid to a 250 volumetric flask.
2. QS to 250 mL using purified water and mix thoroughly.

G. 5N Phosphoric Acid Solution (250 ml)

Molecular weight of phosphoric acid= 98 x 5N = 490 x 250 ml = 122500 mg

1. Add 122.5 g phosphoric acid to a 250 ml volumetric flask.
2. QS to 250 ml using purified water and mix thoroughly.

H. 5N Nitric Acid Solution (250 ml)

Molecular weight of nitric acid = 63.01 x 5N = 315.05 x 250 ml = 78762.5 mg

1. Add 78.76 g nitric acid to a 250 ml volumetric flask.
2. QS to 250 ml using purified water and mix thoroughly.

I. 5N Sulfuric Acid Solution (250 ml)

Molecular weight of sulfuric acid = 98.08 x 5N = 490.4 x 250 ml = 122600 mg

1. Add 122.6 g sulfuric acid to a 250 ml volumetric flask.
2. QS to 250 ml using purified water and mix thoroughly.

J. 5N Ascorbic Acid Solution (250 ml)

Molecular weight of ascorbic acid = 176.12 x 5N = 880.6 x 250 ml = 220150 mg

1. Add 220.15 g ascorbic acid to a 250 ml volumetric flask.
2. QS to 250 ml using purified water and mix thoroughly.

II. Rapastinel 150 mg/ml Preparation

Rapastinel, 150 mg/ml, solutions will be prepared at 250 ml each. The theoretical quantity of rapastinel needed is 37.5 g per 250 ml solution. The actual weight of rapastinel will be adjusted based on potency, residual moisture, total impurities, residualsolvents, and residue on ignition (Table 12).

**Table 12. Data for Adjustment of Total Quantity of Rapastinel.**

| Total Impurities | Water Content(%) | Residual Solvents | Residue on Ignition | Potency(%) |
|---|---|---|---|---|
| 1.52 | 3.3 | 0.3632 | NA | 99.1 |

100 + 1.52+3.3+0.3632 = 105.18 37.5 g X 1.0518 = 39.44 g; Adjust for potency: 100- 99.1 = 0.9; 39.44 x 1.009 = 39.79 g Rapastinel needed per 250 ml batch.

1. Prepare 10 different Rapastinel, 150 mg/ml solutions by dissolving 39.79 g of Rapastinel in approximately 180 ml purified water.
2. Test and record the pH of the drug solution.
3. Adjust the pH of each solution to 4.5 using a different acid for each batch.

    a. Add the acid incrementally and record the volume of acid added at each increment.
    b. Record the pH of the solution after each addition of acid.

4. Mix the solution for 1 hour after reaching pH 4.5.
5. Test and record the pH after 1 hour.
6. Adjust the pH to 4.5 if needed using the respective acid for the solution. Record the volume of acid added and the final pH.
7. QS the solution to 250 ml using purified water, mix well, and record the pH.
8. Filter each solution through a vacuum filtration system with 0.2 1-lm filter.
9. Submit one sample of each solution as T=0 samples for HPLC assays.
10. Store each solution at 40°C and remove one sample from each batch after storage for 2 weeks and 4 weeks.

Freeze the 2 week and 4 week samples until they can be tested together.
11. Samples are tested for pH, appearance, assay / related substances, color, deliverable volume, particle size distribution (HIAC), osmolality, and optical rotation.

EQUIVALENTS

Those skilled in the art will recognize, or be able to ascertain using no more than routine experimentation, many equivalents to the specific embodiments of the invention described herein. Such equivalents are intended to be encompassed by the following embodiments. The issued patents, applications, and references that are cited herein are hereby incorporated by reference to the same extent as if each was specifically and individually incorporated by reference. In the case of inconsistencies, the present disclosure, including definitions, will control. In addition, the materials, methods, and examples are illustrative only and are not intended to be limiting.

Embodiments:

1. A stable, aqueous composition suitable for intravenous injection, comprising:
60 mg/mL to about 200 mg/mL of a pharmaceutically active compound having the formula:

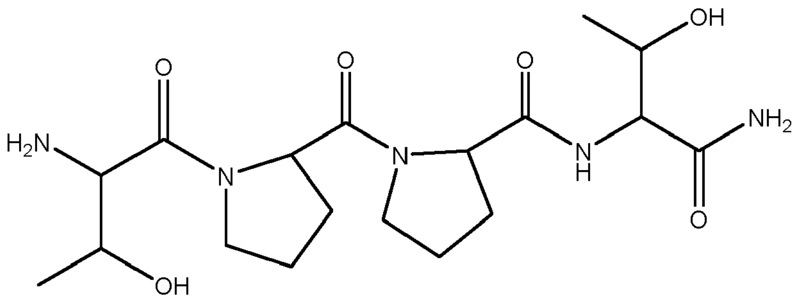

;

or a pharmaceutically acceptable salt thereof;
water for injection; and
an acid; wherein the stable, aqueous composition has a pH of from about 3.9 to about 5.5 at 25 °C.

2. The stable, aqueous composition of embodiment 1, comprising from about 125 mg/mL to about 175 mg/mL of the pharmaceutically active compound.

3. The stable, aqueous composition of embodiment 1 or 2, comprising about 150 mg/mL of the pharmaceutically active compound.

4. The stable, aqueous composition of embodiment 1, comprising about 75 mg/mL of the pharmaceutically active compound.

5. The stable, aqueous composition of any one of embodiments 1-3, comprising about 200 mg to about 500 mg of the pharmaceutically active compound.

6. The stable, aqueous composition of any one of embodiments 1-4, comprising about 450 mg of the pharmaceutically active compound.

7. The stable, aqueous composition of any one of embodiments 1-4, comprising about 375 mg of the pharmaceutically active compound.

8. The stable, aqueous composition of any one of embodiments 1-4, comprising about 225 mg of the pharmaceutically active compound.

9. The stable, aqueous composition of any one of embodiments 1-8, wherein the stable, aqueous composition has a pH of about 4.5 at 25 °C.

10. The stable, aqueous composition of any one of embodiments 1-9 comprising at least one of: $H^+$, a protonated form of the pharmaceutically active compound, and/or a combination thereof.

11. The stable, aqueous composition cof any one of embodiments 1-10, wherein the acid is selected from the group consisting of fumaric acid, malic acid, lactic acid, hydrochloric acid, hydrobromic acid, acetic acid, citric acid, phosphoric acid, nitric acid, sulfuric acid, and ascorbic acid.

12. The stable, aqueous composition cof any one of embodiments 1-11, wherein the acid provides chloride ions in the aqueous composition.

13. The stable, aqueous composition cof any one of embodiments 1-12, wherein the acid is hydrochloric acid.

14. The stable, aqueous composition of any one of embodiments 1-13, wherein upon administration of a dose of the stable, aqueous liquid composition that comprises about 150 mg/mL of the pharmaceutically active compound and has a volume of about 3 mL to a patient, a physiological osmolality of from about 800 mOsmol/kg to about 900 mOsmol/kg is obtained in said patient.

15. The stable, aqueous composition of any one of embodiments 1-13, upon administration of a dose of the stable, aqueous liquid composition that comprises about 75 mg/mL of the pharmaceutically active compound and has a volume of about 3 mL to a patient, a physiological osmolality of from about 375 mOsmol/kg to about 475 mOsmol/kg is obtained in said patient.

16. The stable, aqueous composition of any one of embodiments 1-15, wherein the composition has a minimal amount of one or more of degradation products each selected from the group consisting of cyclo proline-threonine (diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature or after 20 days at room temperature.

17. The stable, aqueous composition of any one of embodiments 1-16, wherein the composition has minimal amounts of one or more of degradation products each selected from the group consisting of diketopiperazine, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 month at 0 °C or below

18. The stable, aqueous composition of any one of embodiments 1-17, wherein the composition has less than about 2% area obtained by HPLC of the GLYX -13 peak of diketopiperazine and/or Pro-Thr-$NH_2$ after 3 months at 40 °C.

19. The stable, aqueous composition of any one of embodiments 1-18, wherein the composition has less than about 1% or less than about 0.5% area obtained by HPLC of the GLYX -13 peak by HPLC of diketopiperazine and/or Pro-Thr-$NH_2$ after 3 weeks at 40 °C.

20. A receptacle containing an amount of the stable, aqueous composition of any one of embodiments 1-19, extractable as at least one single dose.

21. The receptacle of embodiment 20, wherein the single dose has a volume of about 1 mL to about 4 mL.

22. The receptacle of embodiment 20, wherein the single dose has a volume of about 3 mL.

23. A pre-filled syringe comprising a single dose of the stable, aqueous liquid composition of any one of embodiments 1-19.

24. The pre-filled syringe of embodiment 23, wherein the single dose has a volume of about 1 mL to about 4 mL.

25. The pre-filled syringe of embodiment 23, wherein the single dose has a volume of about 3 mL.

26. A composition comprising:
about 150 mg/mL of a compound represented by:

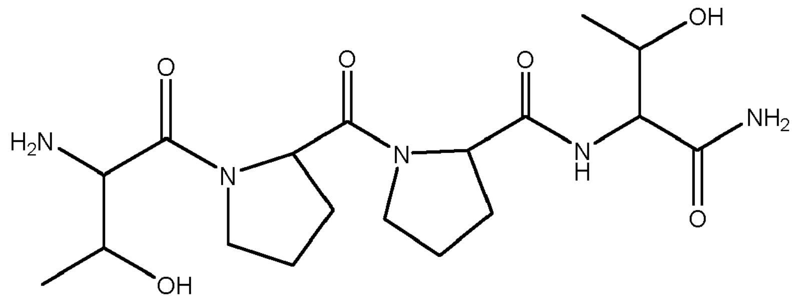

;

water for injection; and
and hydrochloric acid, wherein the composition has a pH of about 4.1 to about 4.7 at 25 °C.

27. A pharmaceutically acceptable dose suitable for injection comprising:
about 450 mg of a compound represented by:

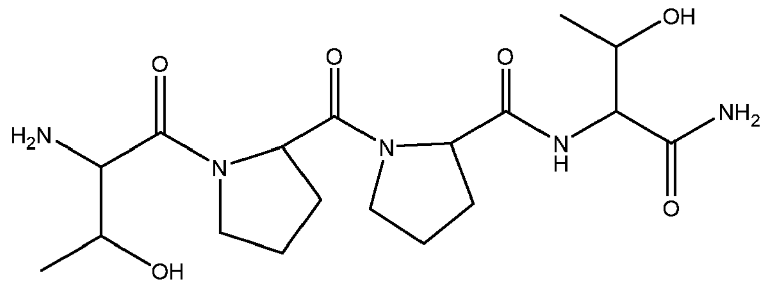

;

water; and
an acid providing chloride ions in the aqueous composition, wherein the dose has a pH of about 4.5 and a volume of about 3 mL.

28. A pharmaceutically acceptable dose suitable for injection comprising:
about 225 mg of a compound represented by:

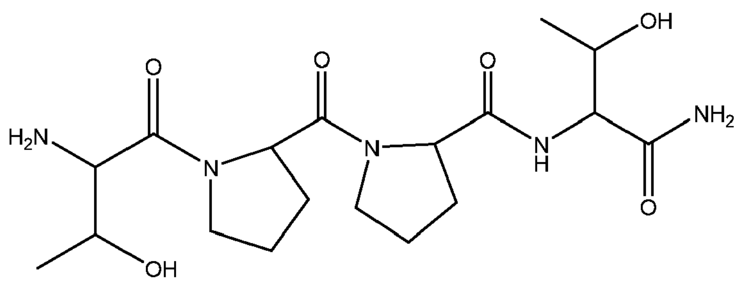

;

water for injection; and
hydrochloric acid, wherein the does has a pH of about 4.5 and a volume of about 3 mL.

29. The dose of embodiments 27 or 28, wherein the dose is disposed within a syringe or a vial.

30. A prefilled syringe or vial comprising a stable, aqueous composition of any one of embodiments 1-19.

31. The composition according to any one of embodiments 1-19, wherein the composition is prepared by a process comprising:

(i) providing a first combination comprising the pharmaceutically active compound and water; and

(ii) contacting the first combination with hydrochloric acid, or a source thereof, in an amount sufficient to achieve a pH of from about 3.9 to about 5.5.

32. A method of treating depression in a patient in need thereof, comprising administering an effective amount of a composition of any one of embodiments 1-19.

33. The method of embodiment 32, wherein depression is refractory depression.

## Claims

1. A stable, aqueous composition suitable for intravenous injection, comprising:
60 mg/mL to about 200 mg/mL of a pharmaceutically active compound having the formula:

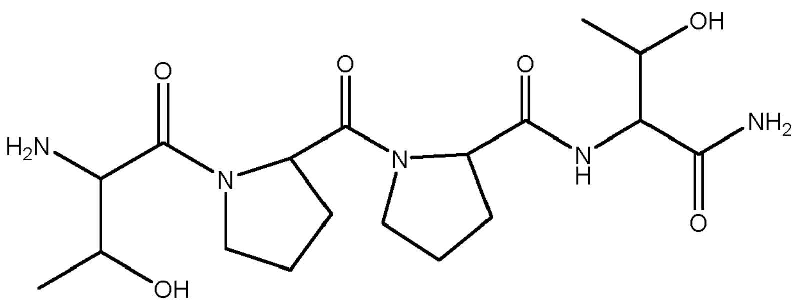
;

or a pharmaceutically acceptable salt thereof;
water for injection; and
an acid; wherein the stable, aqueous composition has a pH of from about 3.9 to about 5.5 at 25 °C.

2. The stable, aqueous composition of claim 1, comprising from about 75 mg/mL, 150 mg/mL, or from about 125 mg/mL to about 175 mg/mL of the pharmaceutically active compound.

3. The stable, aqueous composition of any one of claims 1-2, comprising about 200 mg to about 500 mg of the pharmaceutically active compound, preferably about 225 mg, 375 mg or 450 mg of the pharmaceutically active compound.

**4.** The stable, aqueous composition of any one of claims 1-3, wherein the stable, aqueous composition has a pH of about 4.5 at 25 °C.

**5.** The stable, aqueous composition of any one of claims 1-4 comprising at least one of: $H^+$, a protonated form of the pharmaceutically active compound, and/or a combination thereof.

**6.** The stable, aqueous composition of any one of claims 1-5, wherein the acid is selected from the group consisting of fumaric acid, malic acid, lactic acid, hydrochloric acid, hydrobromic acid, acetic acid, citric acid, phosphoric acid, nitric acid, sulfuric acid, and ascorbic acid, preferably hydrochloric acid.

**7.** The stable, aqueous composition of any one of claims 1-6, wherein the acid provides chloride ions in the aqueous composition.

**8.** The stable, aqueous composition of any one of claims 1-7, wherein upon administration of a dose of the stable, aqueous liquid composition that comprises about 150 mg/mL of the pharmaceutically active compound and has a volume of about 3 mL to a patient, a physiological osmolality of from about 800 mOsmol/kg to about 900 mOsmol/kg is obtained in said patient.

**9.** The stable, aqueous composition of any one of claims 1-7, upon administration of a dose of the stable, aqueous liquid composition that comprises about 75 mg/mL of the pharmaceutically active compound and has a volume of about 3 mL to a patient, a physiological osmolality of from about 375 mOsmol/kg to about 475 mOsmol/kg is obtained in said patient.

**10.** The stable, aqueous composition of any one of claims 1-9, wherein the composition has a minimal amount of one or more of degradation products each selected from the group consisting of cyclo proline-threonine (diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature, after 20 days at room temperature or after 1 month at 0 °C or below.

**11.** The stable, aqueous composition of any one of claims 1-10, wherein the composition has less than about 2% area obtained by HPLC of the GLYX -13 peak of diketopiperazine and/or Pro-Thr-$NH_2$ after 3 months at 40 °C, preferably less than about 1% or less than about 0.5% area obtained by HPLC of the GLYX -13 peak by HPLC of diketopiperazine and/or Pro-Thr-NH2 after 3 weeks at 40 °C.

**12.** A receptacle containing an amount of the stable, aqueous composition of any one of claims 1-11, extractable as at least one single dose.

**13.** The receptacle of claim 12, wherein the single dose has a volume of about 1 mL to about 4 mL, and preferably has a volume of about 3 mL.

**14.** A pre-filled syringe or vial comprising a single dose of the stable, aqueous liquid composition of any one of claims 1-13.

**15.** A stable, aqueous composition according to any one of claims 1-11 comprising:
about 150 mg/mL of a compound represented by:

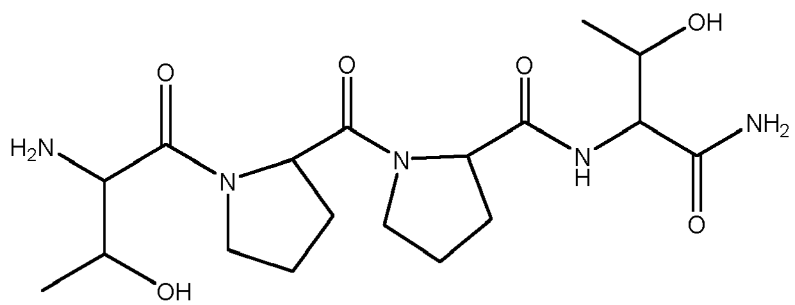

;

water for injection; and
and hydrochloric acid, wherein the composition has a pH of about 4.1 to about 4.7 at 25°C.

**16.** A pharmaceutically acceptable dose suitable for injection comprising:
about 225 mg or 450 mg of a compound represented by:

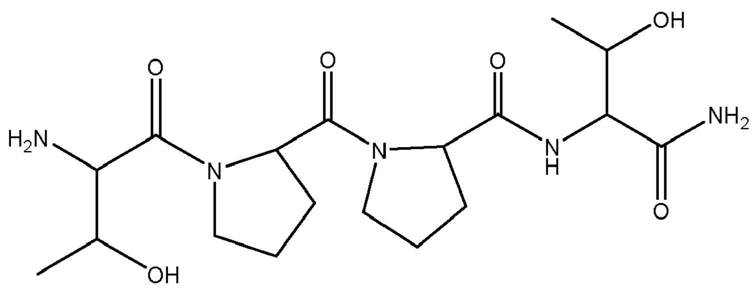

;

water; and
an acid providing chloride ions in the aqueous composition, wherein the dose has a pH of about 4.5 and a volume of about 3 mL.

**17.** The dose of claim 16, wherein the dose is disposed within a syringe or a vial.

**18.** The composition according to any one of claims 1-11 or 15, wherein the composition is prepared by a process comprising:

(i) providing a first combination comprising the pharmaceutically active compound and water; and
(ii) contacting the first combination with hydrochloric acid, or a source thereof, in an amount sufficient to achieve a pH of from about 3.9 to about 5.5.

**19.** The composition according to any one of claims 1-11 or 15 for the use of treating depression in a patient in need thereof.

**Figure 1. shows the formation of Impurity 1 (% area proline-threonine diketopiperazine, RRT 0.43) over time versus pH.**

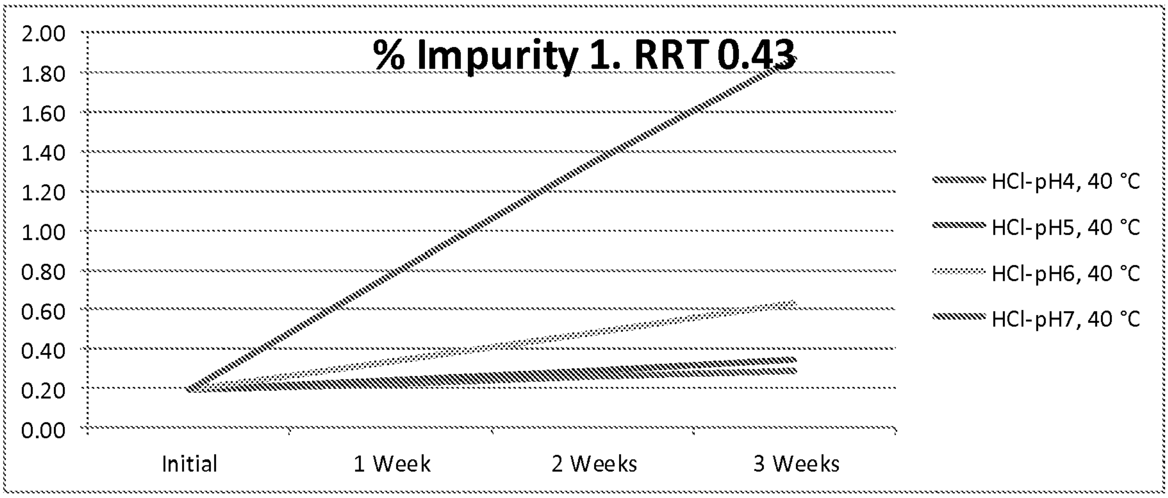


**Figure 2. Formation of Impurity 2 (% area proline-threonine-amide, RRT 0.57) over time versus pH.**

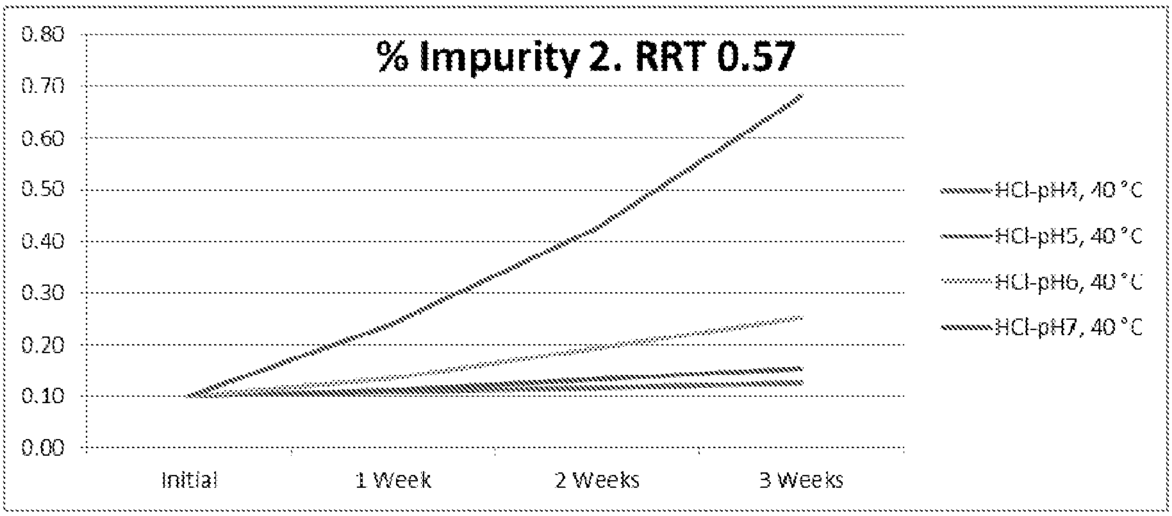

Europäisches Patentamt
European Patent Office
Office européen des brevets

# EUROPEAN SEARCH REPORT

Application Number
EP 20 15 1685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JOSEPH R MOSKAL ET AL: "GLYX-13, an NMDA receptor glycine site functional partial agonist enhances cognition and produces antidepressant effects without the psychotomimetic side effects of NMDA receptor antagonists", EXPERT OPINION ON INVESTIGATIONAL DRUGS, vol. 23, no. 2, 20 November 2013 (2013-11-20), pages 243-254, XP055380018, UK ISSN: 1354-3784, DOI: 10.1517/13543784.2014.852536 * page 244, column 2, paragraph 3 * * page 248, column 1, paragraph 3 * ----- | 1-19 | INV. A61K38/00 A61P25/00 A61K38/05 A61K38/06 A61K9/00 A61K47/12 A61K47/02 A61K47/18 A61K9/08 A61K47/26 A61K31/4025 |
| A | JEFFREY BURGDORF ET AL: "ACCEPTED ARTICLE PREVIEW - GLYX-13, an NMDA Receptor Glycine-Site Functional Partial Agonist, Induces Antidepressant-Like Effects Without Ketamine-Like Side Effects", INTERNET CITATION, 3 December 2012 (2012-12-03), pages 1-46, XP002691138, Retrieved from the Internet: URL:http://www.nature.com/npp/journal/vaop/naam/pdf/npp2012246a.pdf [retrieved on 2013-01-30] * page 1, paragraph 1 * * page 5, paragraph 3 - page 6, paragraph 3 * * page 19, paragraph 2 * ----- | 1-19 | TECHNICAL FIELDS SEARCHED (IPC) A61K |
| A | WO 2013/188465 A2 (UNIV ARIZONA [US]; OLIVE M FOSTER [US]) 19 December 2013 (2013-12-19) * paragraphs [0007], [0012], [0021], [0044], [0049], [0051], [0059] * ----- -/-- | 1-19 | |

The present search report has been drawn up for all claims

1

| Place of search | Date of completion of the search | Examiner |
|---|---|---|
| Munich | 28 May 2020 | Zellner, Eveline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 15 1685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/100585 A1 (MOSKAL JOSEPH [US]; KHAN AMIN [US]) 18 August 2011 (2011-08-18) * paragraphs [0028], [0029], [0047], [0099], [0115] * ----- | 1-19 | |

TECHNICAL FIELDS SEARCHED (IPC)

The present search report has been drawn up for all claims

1

| Place of search | Date of completion of the search | Examiner |
|---|---|---|
| Munich | 28 May 2020 | Zellner, Eveline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 20 15 1685

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2020

| Patent document cited in search report | Publication date | Patent family member(s) | Publication date |
|---|---|---|---|
| WO 2013188465 A2 | 19-12-2013 | US 2015141462 A1 | 21-05-2015 |
| | | WO 2013188465 A2 | 19-12-2013 |
| WO 2011100585 A1 | 18-08-2011 | AU 2011215704 A1 | 30-08-2012 |
| | | CA 2789331 A1 | 18-08-2011 |
| | | CN 102933226 A | 13-02-2013 |
| | | CN 105037492 A | 11-11-2015 |
| | | EP 2542254 A1 | 09-01-2013 |
| | | ES 2706063 T3 | 27-03-2019 |
| | | IL 221400 A | 31-07-2016 |
| | | JP 5928828 B2 | 01-06-2016 |
| | | JP 6328169 B2 | 23-05-2018 |
| | | JP 2013519683 A | 30-05-2013 |
| | | JP 2016185948 A | 27-10-2016 |
| | | JP 2018119004 A | 02-08-2018 |
| | | RU 2012138710 A | 20-03-2014 |
| | | SG 183265 A1 | 27-09-2012 |
| | | SG 10201501050R A | 29-04-2015 |
| | | SG 10201811584R A | 30-01-2019 |
| | | US 2013053325 A1 | 28-02-2013 |
| | | US 2016115197 A1 | 28-04-2016 |
| | | WO 2011100585 A1 | 18-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61984216 B
- US 5763393 A
- US 4086196 A


### Non-patent literature cited in the description


- The Biochemical Basis of Neuropharmacology, Cooper, Bloom and Roth. Oxford University Press, 1986
- **BLISS ; COLLINGRIDGE.** *Nature,* 1993, vol. 361, 31-39